# EUROPEAN PATENT SPECIFICATION

(11) **EP 3 442 544 B1**
(45) Date of publication and mention of the grant of the patent: **29.05.2024**
(21) Application number: 17783351.4
(22) Date of filing: 17.04.2017
(51) Int. Cl.: A61K 35/12, A61K 35/28, A61K 35/44, C12N 5/0789, C12N 5/10, C12N 15/86, C12N 15/87, A61K 35/545

(54) **ENHANCED GENE DELIVERY METHODS**
VERBESSERTE GENFREISETZUNGSVERFAHREN
MÉTHODES AMÉLIORÉES D'ADMINISTRATION DE GÈNES

(30) Priority: 15.04.2016 US 201662323476 P; 01.10.2016 US 201662403110 P
(43) Date of publication of application: 20.02.2019
(73) Proprietor: Angiocrine Bioscience, Inc., San Diego, CA 92172 (US)
(72) Inventor: FINNEGAN, Paul, William, San Diego, CA 92172 (US); DAVIS, Claude, Geoffrey, Auburn, CA 95602 (US); GINSBERG, Michael, Daniel, San Diego, CA 92172 (US); NOLAN, Daniel, Joseph, San Diego, CA 92172 (US)
(74) Representative: Barker Brettell LLP
(86) International application number: PCT/US2017/027884
(87) International publication number: WO 2017/181168

(56) References cited:
- WO-A1-2014/113415
- WO-A1-2014/165131
- WO-A1-2015/057976
- WO-A2-2008/089448
- US-A1- 2005 037 488
- US-A1- 2007 077 652
- M. SEANDEL ET AL: "Generation of a functional and durable vascular niche by the adenoviral E4ORF1 gene", PROCEEDINGS OF THE NATIONAL ACADEMY OF SCIENCES (PNAS), vol. 105, no. 49, 9 December 2008 (2008-12-09), pages 19288-19293, XP55495348, US ISSN: 0027-8424, DOI: 10.1073/pnas.0805980105

## Description

### CROSS-REFERENCE TO RELATED APPLICATIONS

This application claims the benefit of priority of U.S. Provisional Patent Application No. 62/323,476 filed on April 15, 2016, and U.S. Provisional Patent Application No. 62/403,110 filed on October 1, 2016.

Many of the teachings provided in U.S. Patent No. 8,465,732 can be used in conjunction with the present invention, or can be adapted for use with the present invention.

### BACKGROUND OF THE INVENTION

The adenoviral early 4 (E4) region contains at least 6 open reading frames (ORFs). The entire E4 region has been shown previously to regulate angiogenesis and promote survival of endothelial cells (Zhang et al. (2004), J. Biol. Chem. 279(12): 11760-66). Within the entire E4 region, it is the E4ORF1 sequence that is responsible for these biological effects in endothelial cells (U.S. Patent No. 8,465,732, and Seandel et al. (2008), PNAS, 105(49): 19288-93). It has previously been found that endothelial cells engineered to express E4ORF1 are particularly useful in various co-culture methods - where they can be used to support the expansion of a variety of different cell types that are otherwise difficult to maintain or expand in culture, such as various stem cells, including hematopoietic stem cells (U.S. Patent No. 8,465,732, and Seandel et al. (2008), PNAS, 105(49): 19288-93).

Gene therapy, for example using gene editing technologies, offers a promising potential solution to combat a multitude of genetic disorders and diseases. However, a major hurdle to overcome in this field has been the ability to efficiently genetically modify sufficient numbers of autologous cells for transplantation, and also to modify cells of the type most likely to be therapeutically effective. In the case of gene therapy for hematological disorders, such cell types include hematopoietic stem cells (HSCs) and tissue-specific repopulating stem and progenitor cells. Using gene-correction and other gene therapy techniques directed toward this crucial hematopoietic cell population, several hematological disorders could be more effectively treated. Unfortunately, attempts at gene-correction in these and other cells have thus far proven to be largely inefficient.

Several cell transduction and transfection techniques (such as electroporation) cause significant cell stress and cell damage, often resulting in cell death. As such, often only a proportion of cells that are successfully transduced or transfected survive. This is a particular problem for gene delivery to stem cells, and is also a particular problem in applications that require large quantities of healthy, viable, transduced or transfected cells. WO2008/089448 A2 discloses endothelial cells engineered to express the E4ORF1 gene, and their use as feeder cells for culturing HSCs, in promoting survival and proliferation. (Seandel et al., 2008, PNAS 105:49, 19288-19293) discloses that E4ORF1+ endothelial cells support expansion of human leukemic cells and embryonal carcinoma cells. US 2007/077652 A1 discloses a method of co-culturing HSCs with endothelial cells. WO2014/165151 A1 discloses stem cell culture conditions.

### SUMMARY OF THE INVENTION

The present invention is set out in the appended claims and is based, in part, upon certain new discoveries and procedures that are described further in the "Examples" section of this patent disclosure.

For example, it has now been found that the efficiency of gene delivery to certain "target cells," and/or the yield of successfully transfected or transduced target cells, including stem cells, can be significantly increased when the gene delivery is performed in conjunction with endothelial cell co-culture, for example co-culture with endothelial cells that have been optimized to be cultured *ex vivo,* such as endothelial cells that express the E4ORF1 gene (E4ORF1+ ECs).

To the best of Applicants' knowledge, no effect of endothelial cells, and more specifically E4ORF1+ ECs, on the efficiency of gene delivery to other "target" cell types (e.g. cells co-cultured with E4ORF1+ ECs), and/or the yield of successfully transfected or transduced target cells, and/or on the ability to "rescue" cells damaged during a gene delivery process, has been reported previously. Furthermore, the finding that gene delivery efficiency, and/or yield of successfully transduced or transfected cells, is not adversely affected and may even be increased when the gene delivery method is performed in the presence of endothelial cells, and particularly of E4ORF1+ ECs, appears to be counterintuitive - as one might expect the endothelial cells (for example the E4ORF1+ ECs), to take up genetic material that would otherwise have been taken up by the target cells, thereby significantly reducing the overall efficiency of gene delivery to the target cells and/or significantly reducing the yield of successfully transduced or transfected cells. However, the results presented herein demonstrate that this is not the case. On the contrary, the results presented herein demonstrate that the efficiency of gene delivery to target cells, and/or the yield of successfully transduced or transfected cells, is much greater than would be expected in cultures that also comprise endothelial cells, and particularly E4ORF1 + ECs, than in cultures of the target cells alone - even where the total amount of genetic material delivered to the cultures is the same and the relative proportion of the target cells in the cultures is reduced. Building on these discoveries, the present invention provides certain new and improved methods for gene delivery.

Accordingly, the present invention provides an improved method of genetically modifying CD34+ hematopoietic stem or progenitor cells (HSPCs), the method comprising: (a) co-culturing CD34+ hematopoietic stem or progenitor cells (HSPCs) with E4ORF1+ endothelial cells, and (b) contacting the HSPCs with one or more exogenous nucleic acid molecules, wherein the co-culturing step is commenced either: (i) prior to, concurrently with, or after contacting the HSPCs with the one or more exogenous nucleic acid molecules, thereby genetically modifying the CD34+ HSPCs.

In some of such embodiments the gene delivery is part of a gene editing process, and may also involve contacting the target cells with one or more molecules useful in gene editing.

In some such embodiments the target cells are co-cultured with the endothelial cells (e.g. they are present in the same vessel with and/or are in contact with) the target cells at the time that the target cells are contacted with one or more exogenous nucleic acid molecules and/or other molecules.

In some such embodiments the target cells are co-cultured with the endothelial cells for at least 12 hours, or at least 24 hours (1 day), or at least 36 hours, or at least 48 hours (2 days), or at least 60 hours, or at least 72 hours (3 days), or at least 84 hours, or at least 96 hours (4 days) prior to contacting the target cells with one or more exogenous nucleic acid molecules and/or other molecules.

In some such embodiments co-culture of the target cells with the endothelial cells is commenced "immediately" after contacting the target cells with one or more exogenous nucleic acid molecules- in order to quickly "rescue" the target cells from any damage inflicted during the gene delivery process. The term "immediately" in this context, means within 30 minutes of completion of the "contacting" step. In some embodiments co-culture of the target cells with the endothelial cells is commenced within 15 minutes, or within 10 minutes, or within 5 minutes, or within 3 minutes, or within 2 minutes, or within 1 minute, of completion of the "contacting" step. The timing of completion of the "contacting" step will vary depending on the gene delivery method used. For example, when gene delivery is accomplished by electroporation, the "contacting" step is completed when the electrical pulse ends, and the target cells should be placed into co-culture with the endothelial cells after the electrical pulse ends. Similarly, when gene delivery is accomplished by contacting the target cells with a lipofection agent, the "contacting" step is completed when the lipofection agent is removed (e.g. by media replacement), and the target cells should be placed into co-culture with the endothelial cells after removal of the lipofection agent. In some such embodiments the target cells continue to be co-cultured with the endothelial cells for at least 2 hours, at least 6 hours, at least 12 hours, at least 24 hours (1 day), or at least 48 hours (2 days), or at least 72 hours (3 days), or at least 96 hours (4 days), or at least 120 hours (5 days), or at least 144 hours (6 days), or at least 168 hours (7 days), or at least 192 hours (8 days), or at least 240 hours (10 days), or at least 288 hours (12 days) after the contacting step is completed.

In some embodiments ratio of the target cells to the endothelial cells during the co-culturing process is about 1:1. In some embodiments ratio of the target cells to the endothelial cells during the co-culturing process is from about 2:1 t to about 1:2. In some embodiments, the target cell to endothelial cell ratio is about 10:1, or about 9:1, or about 8:1, or about 7:1, or about 6:1, or about 5:1, or about 4:1, or about 3:1, or about 2:1, or about 1:2, or about 1:3, or about 1:4, or about 1:5, or about 1:6, or about 1:7, or about 1:8, or about 1:9, or about 1:10. In some such embodiments, these are the ratios of target cells to the endothelial cells and the time the co-culture is commenced. In some such embodiments, these are the ratios of target cells to the endothelial cells and the time the targets cells are contacted with the one or more exogenous nucleic acid molecules and/or other molecules.

In some embodiments the methods provided herein result in a target cell transduction or transfection efficiency of greater than about 10%. In some embodiments the methods provided herein result in a target cell transduction or transfection efficiency of greater than about 20%. In some embodiments the methods provided herein result in a target cell transduction or transfection efficiency of greater than about 30%. In some embodiments the methods provided herein result in a target cell transduction or transfection efficiency of greater than about 40%. In some embodiments the methods provided herein result in a target cell transduction or transfection efficiency of greater than about 50%. In some embodiments the methods provided herein result in a target cell transduction or transfection efficiency of greater than about 60%. In some embodiments the methods provided herein result in a target cell transduction or transfection efficiency of greater than about 65%. In some embodiments the methods provided herein result in a target cell transduction or transfection efficiency of greater than about 70%. In some embodiments the methods provided herein result in a target cell transduction or transfection efficiency of greater than about 75%. In some embodiments the methods provided herein result in a target cell transduction or transfection efficiency of greater than about 80%. In some embodiments the methods provided herein result in a target cell transduction or transfection efficiency of greater than about 85%. In some embodiments the methods provided herein result in a target cell transduction or transfection efficiency of greater than about 90%. In some embodiments the methods provided herein result in a target cell transduction or transfection efficiency of greater than about 95%.

In some embodiments the methods provided herein result in a target cell transduction or transfection efficiency that is greater than that obtained in the absence of the endothelial cell co-culture. In some embodiments the methods provided herein result in a target cell transduction or transfection efficiency that is about 10% greater than that obtained in the absence of the endothelial cell co-culture. In some embodiments the methods provided herein result in a target cell transduction or transfection efficiency that is about 20% greater than that obtained in the absence of the endothelial cell co-culture. In some embodiments the methods provided herein result in a target cell transduction or transfection efficiency that is about 30% greater than that obtained in the absence of the endothelial cell co-culture. In some embodiments the methods provided herein result in a target cell transduction or transfection efficiency that is about 40% greater than that obtained in the absence of the endothelial cell co-culture. In some embodiments the methods provided herein result in a target cell transduction or transfection efficiency that is about 50% greater than that obtained in the absence of the endothelial cell co-culture. In some embodiments the methods provided herein result in a target cell transduction or transfection efficiency that is about 60% greater than that obtained in the absence of the endothelial cell co-culture. In some embodiments the methods provided herein result in a target cell transduction or transfection efficiency that is about 70% greater than that obtained in the absence of the endothelial cell co-culture. In some embodiments the methods provided herein result in a target cell transduction or transfection efficiency that is about 80% greater than that obtained in the absence of the endothelial cell co-culture. In some embodiments the methods provided herein result in a target cell transduction or transfection efficiency that is about 90% greater than that obtained in the absence of the endothelial cell co-culture. In some embodiments the methods provided herein result in a target cell transduction or transfection efficiency that is about 100% greater than that obtained in the absence of the endothelial cell co-culture. In some embodiments the methods provided herein result in a target cell transduction or transfection efficiency that is about 150% greater than that obtained in the absence of the endothelial cell co-culture. In some embodiments the methods provided herein result in a target cell transduction or transfection efficiency that is about 200% greater than that obtained in the absence of the endothelial cell co-culture. In some embodiments the methods provided herein result in a target cell transduction or transfection efficiency that is about 250% greater than that obtained in the absence of the endothelial cell co-culture. In some embodiments the methods provided herein result in a target cell transduction or transfection efficiency that is about 300% greater than that obtained in the absence of the endothelial cell co-culture.

In some embodiments the methods provided herein result in a target cell transduction or transfection efficiency that is similar to that obtained in the absence of the endothelial cell co-culture, even under conditions where one would have expected a reduction in the transfection or transduction efficiency because, for example, the amount of the exogenous nucleic acid molecules supplied to the target cells co-cultured with endothelial cells and to the target cells not co-cultured with endothelial cells is the same (or about the same), and/or because the amount of the exogenous nucleic acid molecules available to transfect or transduce the target cells is reduced because of the presence of the co-cultured endothelial cells.

In some embodiments the methods provided herein result in a target cell transduction or transfection efficiency that is reduced by only about 10%, or about 20%, or about 30%, or about 40%, or about 50% as compared to that obtained in the absence of the endothelial cell co-culture, even under conditions where one would have expected a much greater reduction in the transfection or transduction efficiency because, for example, the amount of the exogenous nucleic acid molecules supplied to the target cells co-cultured with endothelial cells and to the target cells not co-cultured with endothelial cells is the same (or about the same), and/or because the amount of the exogenous nucleic acid molecules available to transfect or transduce the target cells is reduced significantly because of the presence of the co-cultured endothelial cells.

In some embodiments the methods provided herein result in a yield of successfully transfected or transduced target cells that is greater than that obtained in the absence of the endothelial cell co-culture. In some embodiments the methods provided herein result in a yield of successfully transfected or transduced target cells that is about 10% greater than that obtained in the absence of the endothelial cell co-culture. In some embodiments the methods provided herein result in a yield of successfully transfected or transduced target cells that is about 20% greater than that obtained in the absence of the endothelial cell co-culture. In some embodiments the methods provided herein result in a yield of successfully transfected or transduced target cells that is about 30% greater than that obtained in the absence of the endothelial cell co-culture. In some embodiments the methods provided herein result in a yield of successfully transfected or transduced target cells that is about 40% greater than that obtained in the absence of the endothelial cell co-culture. In some embodiments the methods provided herein result in a yield of successfully transfected or transduced target cells that is about 50% greater than that obtained in the absence of the endothelial cell co-culture. In some embodiments the methods provided herein result in a yield of successfully transfected or transduced target cells that is about 60% greater than that obtained in the absence of the endothelial cell co-culture. In some embodiments the methods provided herein result in a yield of successfully transfected or transduced target cells that is about 70% greater than that obtained in the absence of the endothelial cell co-culture. In some embodiments the methods provided herein result in a yield of successfully transfected or transduced target cells that is about 80% greater than that obtained in the absence of the endothelial cell co-culture. In some embodiments the methods provided herein result in a yield of successfully transfected or transduced target cells that is about 90% greater than that obtained in the absence of the endothelial cell co-culture. In some embodiments the methods provided herein result in a yield of successfully transfected or transduced target cells that is about 100% greater than that obtained in the absence of the endothelial cell co-culture. In some embodiments the methods provided herein result in a yield of successfully transfected or transduced target cells that is about 150% greater than that obtained in the absence of the endothelial cell co-culture. In some embodiments the methods provided herein result in a yield of successfully transfected or transduced target cells that is about 200% greater than that obtained in the absence of the endothelial cell co-culture. In some embodiments the methods provided herein result in a yield of successfully transfected or transduced target cells that is about 250% greater than that obtained in the absence of the endothelial cell co-culture. In some embodiments the methods provided herein result in a yield of successfully transfected or transduced target cells that is about 300% greater than that obtained in the absence of the endothelial cell co-culture. In some embodiments the methods provided herein result in a yield of successfully transfected or transduced target cells that is about 400% greater than that obtained in the absence of the endothelial cell co-culture. In some embodiments the methods provided herein result in a yield of successfully transfected or transduced target cells that is about 500% greater than that obtained in the absence of the endothelial cell co-culture. In some embodiments the methods provided herein result in a yield of successfully transfected or transduced target cells that is about 600% greater than that obtained in the absence of the endothelial cell co-culture. In some embodiments the methods provided herein result in a yield of successfully transfected or transduced target cells that is more than 600% greater than that obtained in the absence of the endothelial cell co-culture.

In some embodiments, the co-culturing step of such gene delivery methods is performed under atmospheric oxygen conditions. In some embodiments, the co-culturing step of such gene delivery methods is performed under normoxic conditions. In some embodiments, the co-culturing step of such gene delivery methods is performed under hypoxic conditions. In some embodiments, the co-culturing step of such gene delivery methods is performed under severely hypoxic conditions. In some embodiments, the co-culturing step of such gene delivery methods is performed at oxygen levels ranging from 18% to 0.1% oxygen.

It is expected that the improved gene delivery methods of the present invention may be particularly useful for performing "gene editing" of target cells - for example using site-specific nucleases to deliver "gene-corrected" nucleic acid sequences to specific sites within the genome of the target cells. Thus, in some embodiments the methods described above also comprise contacting the target cells with one or more exogenous nucleases, such as sequence-specific nucleases. Such nucleases include, but are not limited to, meganucleases, zinc finger nucleases, transcription activator-like effector-based nucleases (TALENs), and CRISPR-Cas system nucleases.

The gene delivery methods of the present disclosure can be used to deliver exogenous nucleic acids to, and/or to genetically modify, any desired target cell type. The target cells may be differentiated cells, such as, but not limited to, for example, lymphocytes, T-cells, B-cells, NK cells, myeloid cells, endocrine cells, mesenchymal cell, or epidermal cells. The target cells may be stem cells or progenitor cells, including, but not limited to pluripotent stem cells - such as embryonic stem cells (ES cells) or induced pluripotent stem cells (iPS cells). The stem cells may be tissue- or organ-restricted stem or progenitor cells - i.e. stem or progenitor cells that are committed to a certain cell or tissue lineage. The target cells may be hematopoietic stem cells (HSCs) or hematopoietic stem or progenitor cells (HSPCs). The HSCs or HSPCs are CD34-positive (CD34+). In some embodiments the HSCs or HSPCs are derived from bone-marrow, peripheral blood, umbilical cord blood, amniotic fluid, or other sources of stem cells.

In some embodiments any suitable type of endothelial cells can be used in the gene delivery methods described above and elsewhere herein. In preferred embodiments the endothelial cells and the target cells are derived from same tissue or organ. Typically the endothelial cells are vascular endothelial cells, such as primary vascular endothelial cells, for example umbilical vein endothelial cells or UVEC cells, which is part of the hematopoietic organ system. Preferably the endothelial cells are human endothelial cells, such as human umbilical vein endothelial cells or "HUVECs." Preferably the target cells are hematopoietic stem and/or progenitor cells.

The step of contacting the target cells with one or more exogenous nucleic acid molecules or other molecules useful in gene editing can be carried out using any suitable method known in the art. In some embodiments this step is performed by transfection, for example using a method such as liposome-mediated transfection, polybrene-mediated transfection, DEAE dextran-mediated transfection, electroporation, calcium phosphate precipitation, microinjection, or micro-particle bombardment. In other embodiments this step is performed by transduction with a virus, for example using lentivirus-mediated transduction, adenovirus-mediated transduction, retrovirus-mediated transduction, adeno-associated virus-mediated transduction or herpesvirus-mediated transduction.

The nucleic acid molecule delivered to the target cells using the methods described herein can be any nucleic acid molecule that is desired. For example, the nucleic acid molecule may encode a therapeutically useful protein, or a marker protein, or any other desired protein - without limitation. Similarly, the nucleic acid molecule delivered may not encode a protein. For example, it may encode a fragment of a protein, such as "gene-corrected" fragment of a nucleotide sequence present in the genome of the target cell, for example for use in correcting a genetic defect in the target cell. In some such methods the gene-corrected sequence may be used to replace a mutant sequence in the genome of the target cell, for example using one or more gene-editing / gene-correction technologies, including, but not limited to meganuclease, zinc finger nuclease, TALEN, and/or CRISPR-Cas more gene-editing / gene-correction technologies.

The various methods described above, and elsewhere in this patent disclosure, result in the generation of "genetically modified target cells." These genetically modified target cells can be used as desired. The genetically modified target cells may be administered to a subject in need thereof, such as a human or non-human subject. In some such embodiments the subject may have a disease or disorder affecting the target cells, for example a genetic disease or disorder, and/or a disease or disorder that results in a deficiency of the target cell population. The genetically modified target cells are HSCs or HSPCs and are administered to a subject having a disease or disorder that affects cells of the hematopoietic system. For example, in some embodiments such a subject may have a deficiency in hematopoiesis caused by a myeloablative treatment. In other embodiments such a subject may have a hematologic disease, an infectious immunodeficiency, an infectious disease affecting T cells, a genetic immunodeficiency, severe combined immunodeficiency, a genetic disease affecting erythrocytes, and/or an anemia - such as sickle cell anemia, Fanconi's anemia, or thalassemia. In some such embodiments the target cells may be allogeneic with respect to the subject. In other such embodiments the target cells may be autologous with respect to the subject.

Also disclosed but not part of the invention herein are genetically modified target cells produced using the methods described herein, and/or compositions comprising such genetically modified target cells, for example therapeutic compositions.

### BRIEF DESCRIPTION OF THE DRAWINGS

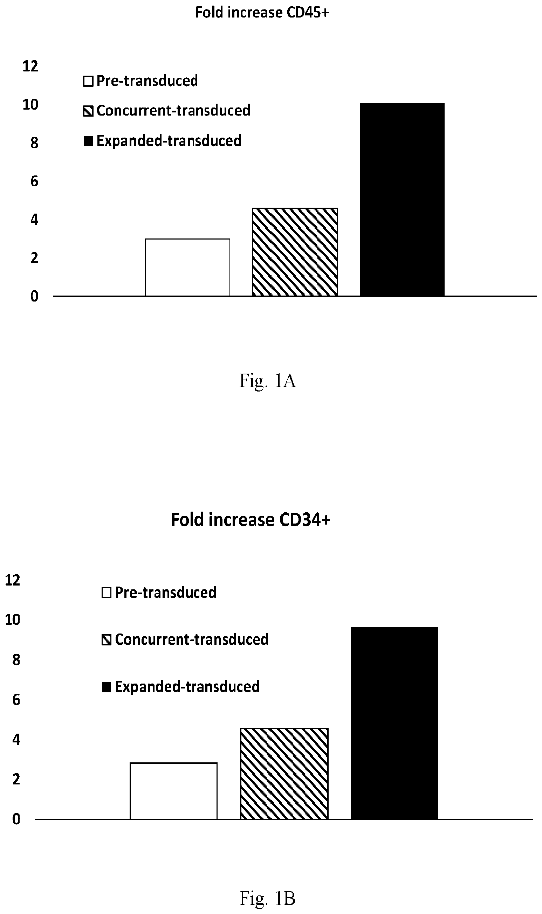

**Fig. 1A-B.** Expansion of CD34+ cells on E4ORF1+ human umbilical vein endothelial cells (UVEC cells) prior to initiating transduction process results in increased fold expansion of total hematopoietic cells and CD34+ cells. **Fig. 1A** - Fold expansion of CD45+ (total) hematopoietic cells after 8 days. **Fig. 1B** - Fold expansion of CD34+ hematopoietic cells after 8 days ("Pre-transduced" samples - white bars. "Concurrent-transduced" samples - grey bars. "Expanded-transduced" samples - black bars). All conditions not specified herein are specified in the Examples.

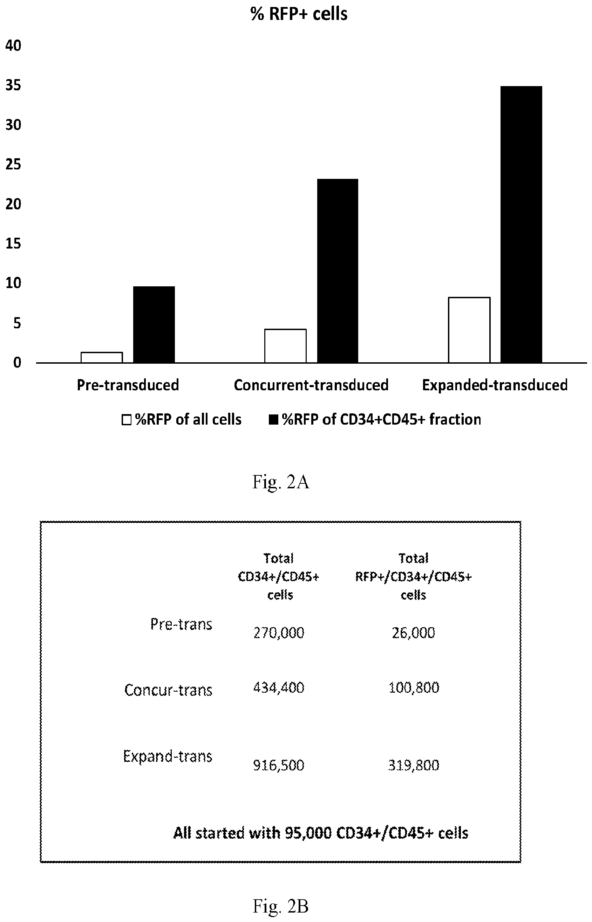

**Fig. 2A-B.** Expansion of CD34+ cells on E4ORF1+ UVEC cells prior to initiating transduction process results in increased transduction efficiency of cell populations. **Fig. 2A** **-** % of Red Fluorescent Protein (RFP)-labeled cells after 8 days (white bars - % of total cell population; black bars - % of CD34+ cell population). **Fig. 2B** - Total CD34+ cells and total RFP+/CD34+ cells after 8 days. All conditions not specified herein are specified in the Examples.

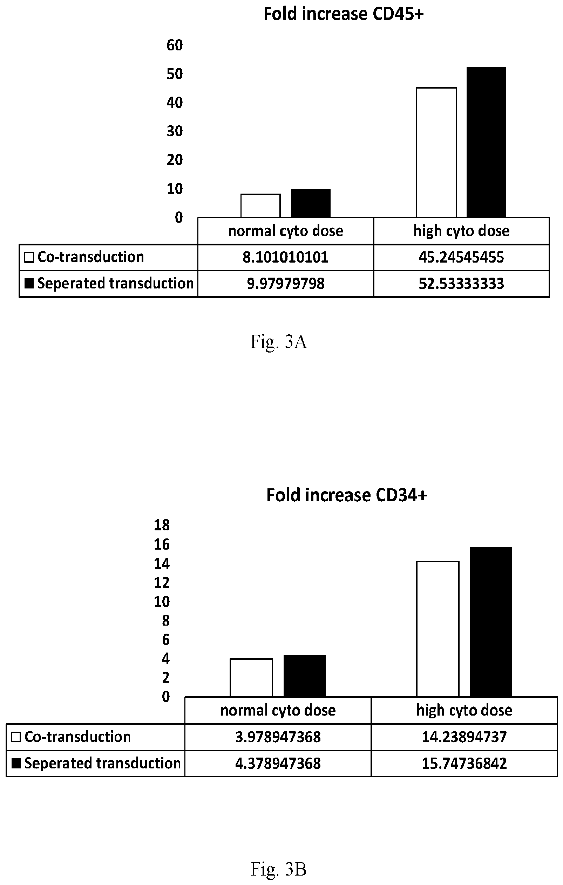

**Fig. 3A-B.** Expansion of CD34+ cells on E4ORF1+ UVEC cells improves with increasing cytokine dose. **Fig. 3A** - Fold expansion of CD45+ (total) hematopoietic cells after 7 days. **Fig. 3B** - Fold expansion of CD34+ hematopoietic cells after 7 days ("Co-transduced" samples - white bars. "Separated-transduced" samples - black bars). All conditions not specified herein are specified in the Examples.

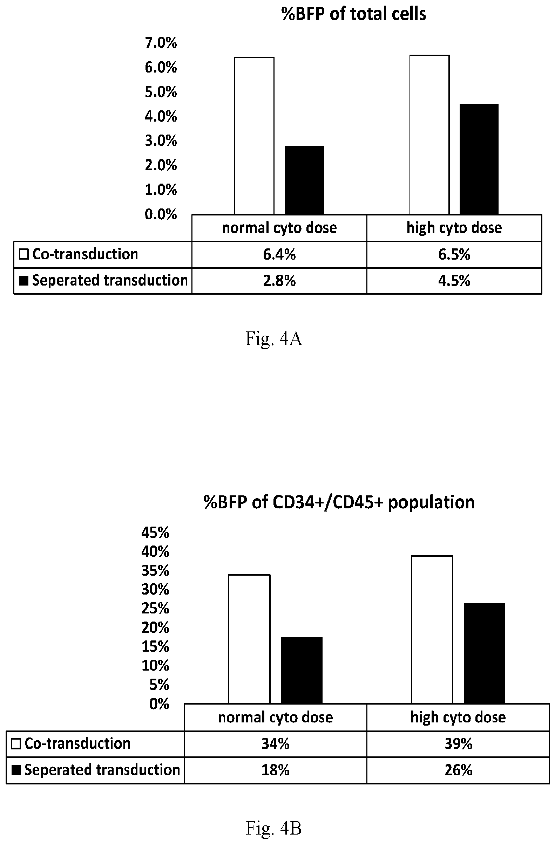

**Fig. 4A-B.** Efficiency of transduction is increased for CD34+ cells when transduction is performed in concert with E4ORF1+ UVEC co-culture. **Fig. 4A** - % BFP-labeled cells after 7 days - total cell population. **Fig. 4B** - % BFP-labeled cells after 7 days - CD34+/CD45+ cell population ("Co-transduced" samples - white bars. "Separated-transduced" samples - black bars). All conditions not specified herein are specified in the Examples.

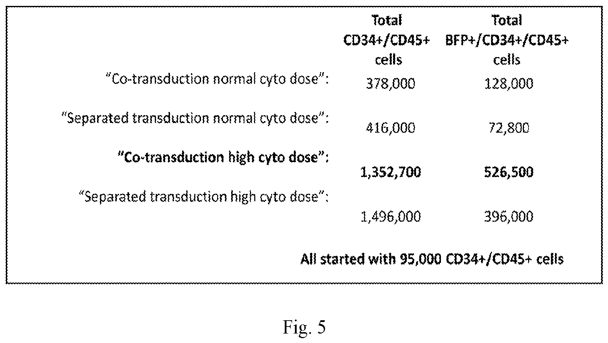

**Fig. 5**. Increasing cytokine dose in combination with transduction of CD34+ cells while in co-culture with E4ORF1+ UVEC cells results in both increased transduction efficiency of and overall cellular yield of transduced CD34+ cell population. All conditions not specified herein are specified in the Examples.

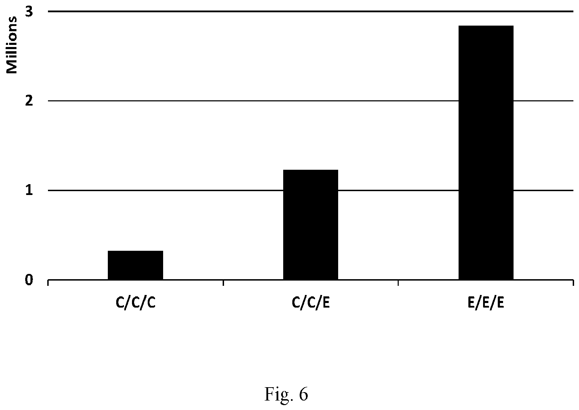

**Fig. 6****.** Total hematopoietic cellular yield is increased during electroporation with the use of E4ORF1+ UVEC cells. 500,000 CD34+ cells isolated from mobilized peripheral blood (mPB) were transfected. The transfection delivered a green fluorescent protein (GFP) expressing plasmid via electroporation. During the stimulation, transfection, and recovery/expansion phases, CD34+ cells were exposed to cytokines (SCF, TPO, Flt3-L) alone (denoted as C/C/C), cytokines during the stimulation and transfection stages followed by recovery in direct co-culture with E4ORF1+ UVEC cells (C/C/E), or in the presence of E4ORF1+ UVEC cells during all 3 stages (E/E/E). Fluorescent antibodies to identify CD45 on hematopoietic cells were used to determine the hematopoietic content of the cultures and combined with hemocytometer counts resulting in the yields presented here (axis shows numbers of cells in millions). All conditions not specified herein are specified in the Examples.

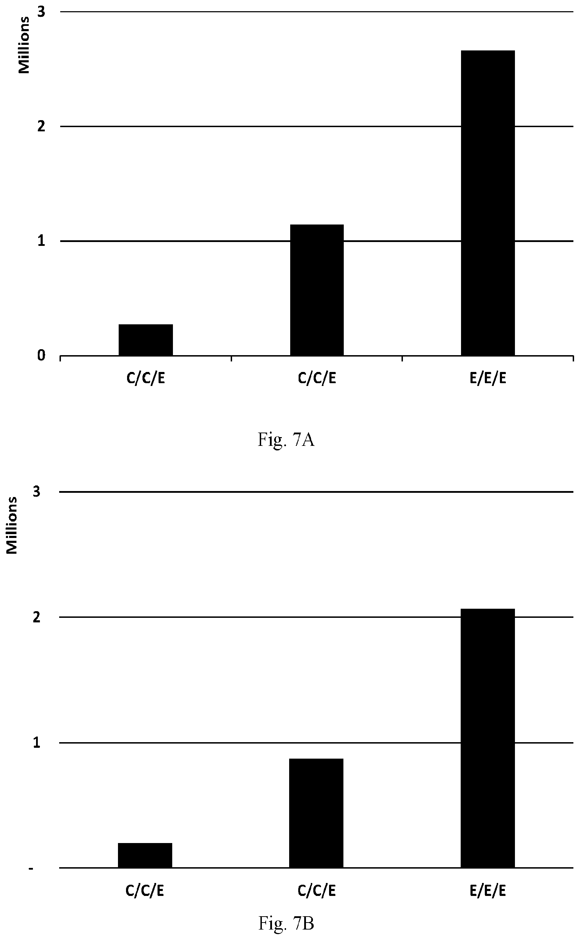

**Fig. 7A-B.** Total CD45+ CD34+ HSPC cell yield is increased during electroporation with the use of E4ORF1+ UVEC cells. 500,000 CD34+ cells isolated from mobilized peripheral blood were transfected. The transfection delivered a GFP-expressing plasmid via electroporation. During the stimulation, transfection, and recovery/expansion phases, CD34+ cells were exposed to cytokines (SCF, TPO, Flt3-L) alone (denoted as C/C/C), cytokines during the stimulation and transfection stages followed by recovery in direct co-culture with E4ORF1+ UVEC cells (C/C/E), and in the presence of E4ORF1+ UVEC cells during all 3 stages (E/E/E). Flow cytometry was used to quantify the number of cells. Fluorescent antibodies to identify CD34 and CD45 on hematopoietic stem and progenitor cells were used to determine the HSPC content of the cultures. GFP expression denoted successfully transfected cells. After quantification via flow cytometry and cell counting using a hemocytometer, total CD45+ CD34+ HSPC yield is presented in the bar graph shown in **Fig. 7A** (axis shows numbers of cells in millions) and successfully transfected CD45+ CD34+ HSPC yield is presented in the bar graph shown in **Fig. 7B** (axis shows numbers of cells in millions). All conditions not specified herein are specified in the Examples.

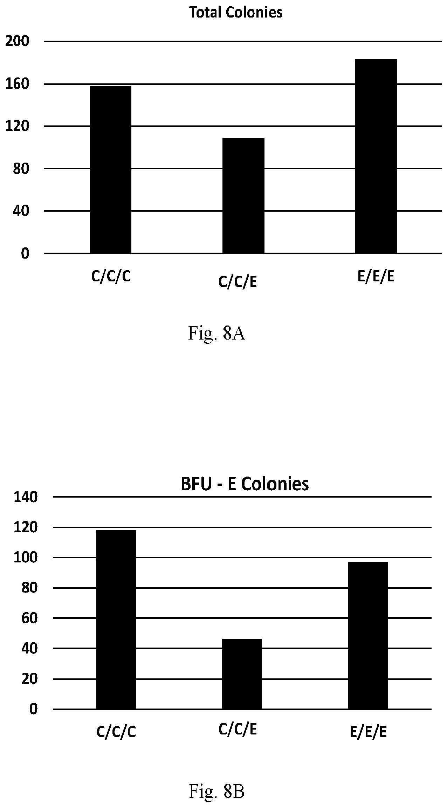

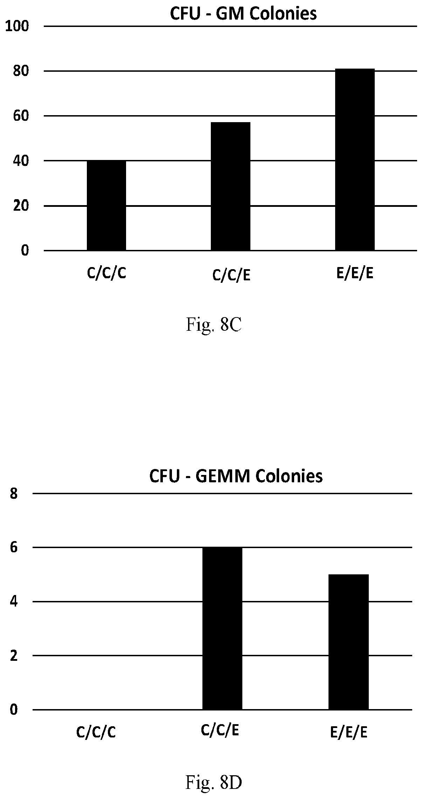

**Fig. 8A-D.** Colony Forming Units (CFUs) are increased when co-culturing HSPCs with E4ORF1+ UVEC cells during electroporation. During the stimulation, transfection, and recovery/expansion phases, CD34+ cells were exposed to cytokines (SCF, TPO, Flt3-L) alone (denoted as C/C/C), cytokines during the stimulation and transfection stages followed by recovery in direct co-culture with E4ORF1+ UVEC cells (C/C/E), and in the presence of E4ORF1+ UVEC cells during all 3 stages (E/E/E), as described in the Examples. For colony forming unit (CFU) assays 1000 total cells (HSPCs alone or HSPCs co-cultured with E4ORF1+ UVEC cells) were plated in two wells of a 6-well dish for each condition in methylcellulose for two weeks. A Stem Vision instrument was used quantify and distinguish the frequency of colonies per each 1000 plated cells. **Fig. 8A** is a bar graph showing quantification of all types of colonies (axis shows total number of colonies). **Fig. 8B** is a bar graph depicting Blast Forming Unit-erythrocyte colonies (BFU-E) (axis shows number of BFU-E colonies). **Fig. 8C** is a bar graph quantifying Colony Forming Units-granulocyte macrophage (CFU-GM) (axis shows number of CFU-GM colonies). **Fig. 8D** is a bar graph quantifying Colony Forming Units-granulocyte erythrocyte macrophage megakaryocyte (CFU-GEMM) (axis shows number of CFU-GEMM colonies). All conditions not specified herein are specified in the Examples.

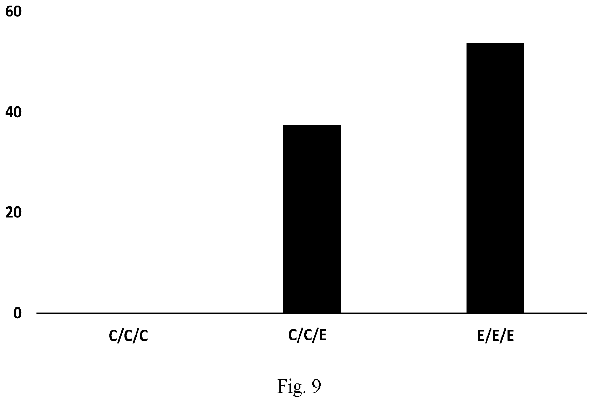

**Fig. 9****.** Colony Forming Units (CFU) are increased when co-culturing HSPCs with E4ORF1+ UVEC cells during electroporation. During the stimulation, transfection, and recovery/expansion phases, CD34+ cells were exposed to cytokines (SCF, TPO, Flt3-L) alone (denoted as C/C/C), cytokines during the stimulation and transfection stages followed by recovery in direct co-culture with E4ORF1+ UVEC cells (C/C/E), and in the presence of E4ORF1+ UVEC cells during all 3 stages (E/E/E), as described in the Examples. For colony forming unit (CFU) assays 1000 total cells (HSPCs alone or HSPCs co-cultured with E4ORF1+ UVEC cells) were plated in two wells of a 6-well dish for each condition in methylcellulose for two weeks. A Stem Vision instrument was used quantify and distinguish the frequency of colonies per each 1000 plated cells. In order to quantify the rate of Colony Forming Units-granulocyte erythrocyte macrophage megakaryocyte (CFU-GEMM), the data was normalized to account for the presence of E4ORF1+ UVEC cells, which do not give rise to colonies. The bar graph represents the frequency of CFU-GEMMs within total expanded product. All conditions not specified herein are specified in the Examples.

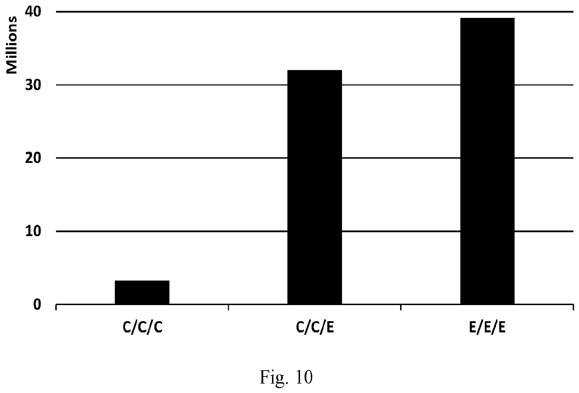

**Fig. 10****.** Total hematopoietic cellular yield is increased during lentiviral transduction with the use of E4ORF1+ UVEC cells. 450,000 CD34+ cells isolated from mobilized peripheral blood were transduced with a multiplicity of infection (MOI) of 25. The transduction delivered a green fluorescent protein (GFP) expressing transgene. During the stimulation, transduction, and recovery/expansion phases, CD34+ cells were exposed to cytokines (SCF, TPO, Flt3-L) alone (denoted as C/C/C), cytokines during the stimulation and transduction stages followed by recovery in direct co-culture with E4ORF1+ UVEC cells (C/C/E), and in the presence of E4ORF1+ UVEC cells during all 3 stages (E/E/E). Fluorescent antibodies to identify CD45 on hematopoietic cells was used to determine the amount of hematopoietic content of the cultures and combined with hemocytometer counts resulted in the yields presented in the bar graph (axis shows numbers of cells in millions). All conditions not specified herein are specified in the Examples.

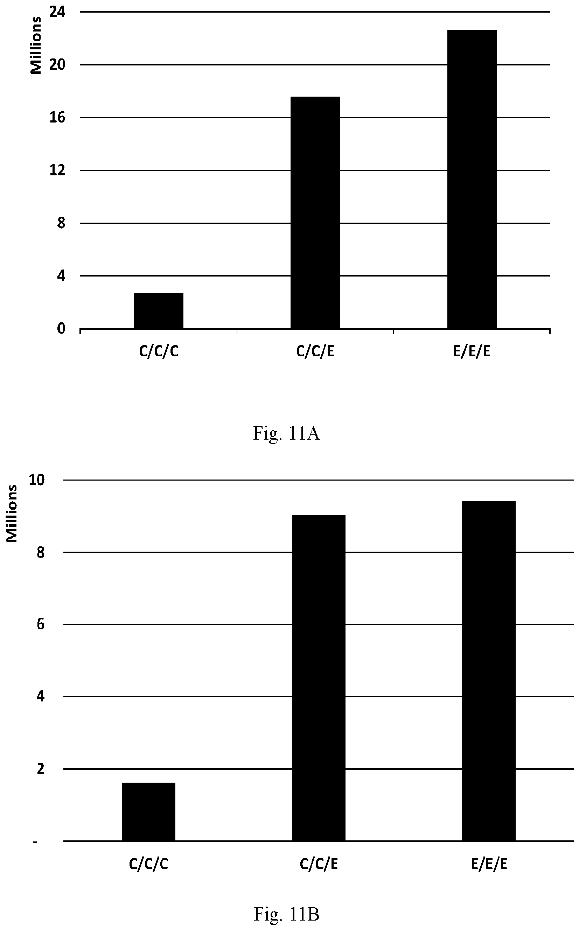

**Fig. 11A-B.** Total CD45+ CD34+ HSPC cellular yield is increased during lentiviral transduction with the use of E4ORF1+ UVEC cells. 450,000 CD34+ cells isolated from mobilized peripheral blood were transduced with a multiplicity of infection (MOI) of 25. The transduction delivered a green fluorescent protein (GFP) expressing transgene. During the stimulation, transduction, and recovery/expansion phases, CD34+ cells were exposed to cytokines (SCF, TPO, Flt3-L) alone (denoted as C/C/C), cytokines during the stimulation and transduction stages followed by recovery in direct co-culture with E4ORF1+ UVEC cells (C/C/E), and in the presence of E4ORF1+ UVEC cells during all 3 stages (E/E/E). Flow cytometry was used to quantify the number of cells. Fluorescent antibodies were used to identify CD34 and CD45 on hematopoietic stem and progenitor cells and determine the HSPC content of the cultures. GFP expression indicated successfully transduced cells. After quantification via flow cytometry and using hemocytometer counts, total CD45+ CD34+ HSPC yield is presented in the bar graph in **Fig. 11A****,** with successfully transfected CD45+ CD34+ HSPC yield presented in the bar graph in **Fig. 11B****.** Axes show numbers of cells in millions. All conditions not specified herein are specified in the Examples.

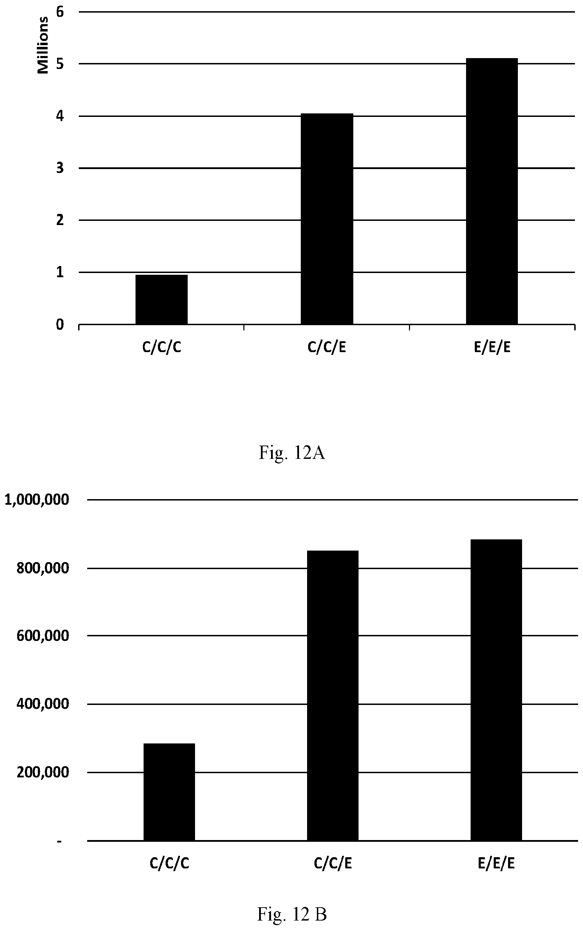

**Fig. 12A-B.** Total CD45+ CD34+ CD38- CD45RA- CD49f+ stem cell yield is increased during lentiviral transduction with the use of E4ORF1+ UVEC cells. 450,000 CD34+ cells isolated from mobilized peripheral blood were transduced with a multiplicity of infection (MOI) of 25. The transduction delivered a GFP-expressing transgene. During the stimulation, transduction, and recovery/expansion phases, CD34+ cells were exposed to cytokines (SCF, TPO, Flt3-L) alone (denoted as C/C/C), cytokines during the stimulation and transduction stages followed by recovery in direct co-culture with E4ORF1+ UVEC cells (C/C/E), and in the presence of E4ORF1+ UVEC cells during all 3 stages (E/E/E). Flow cytometry was used to quantify the number of cells. Fluorescent antibodies to identify CD38, CD45RA, CD49f, CD34 and CD45 on hematopoietic stem cells were used to determine the stem cell content of the cultures. GFP expression denoted successfully transduced cells. After quantification via flow cytometry and using hemocytometer counts, total stem cell yield is presented in the bar graph in **Fig. 12A** (axis shows numbers of cells in millions), with successfully transduced stem cell yield presented in the bar graph in **Fig. 12B** (axis show numbers of cells). All conditions not specified herein are specified in the Examples.

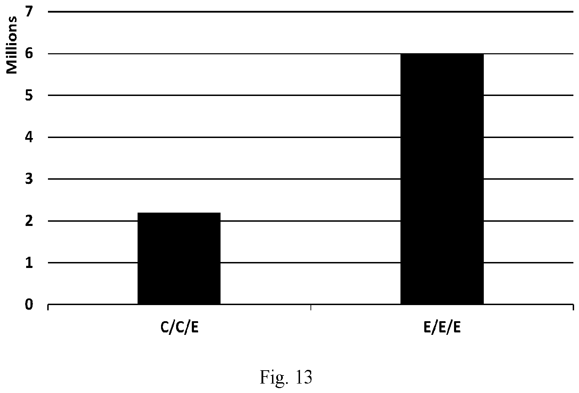

**Fig. 13****.** Total hematopoietic cellular yield is increased during lentiviral transduction with the use of E4ORF1+ UVEC cells. 300,000 CD34+ cells isolated from mobilized peripheral blood were transduced with a multiplicity of infection (MOI) of 50. The transduction delivered a GFP-expressing transgene. During the stimulation, transduction, and recovery/expansion phases, CD34+ cells were exposed to cytokines (SCF, TPO, Flt3-L) during the stimulation and transduction stages followed by recovery in direct co-culture with E4ORF1+ UVEC cells (C/C/E), and in the presence of E4ORF1+ UVEC cells during all 3 stages (E/E/E). Fluorescent antibodies to identify CD45 on hematopoietic cells were used to determine the hematopoietic content of the cultures and combined with hemocytometer counts to determine the cell yields presented in the bar graph (axis shows numbers of cells in millions). All conditions not specified herein are specified in the Examples.

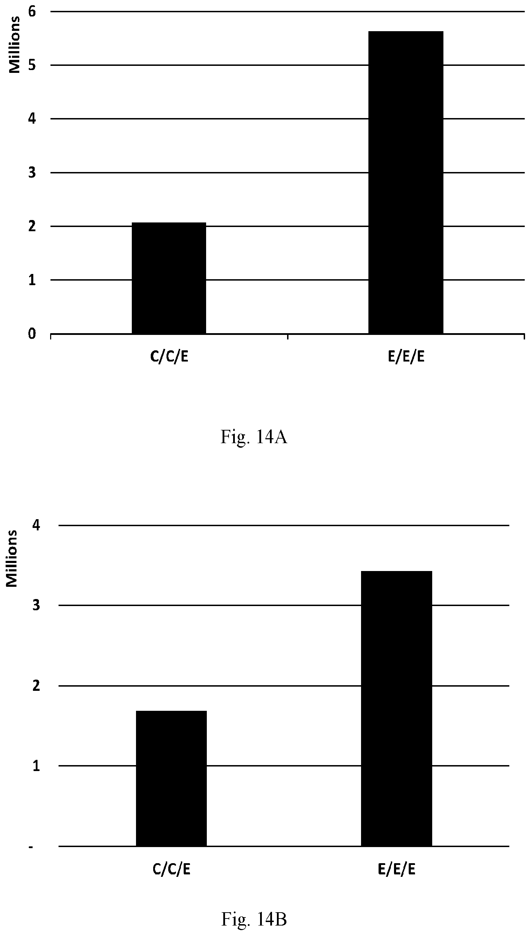

**Fig. 14A-B.** Total CD45+ CD34+ HSPC cellular yield is increased during lentiviral transduction with the use of E4ORF1+ UVEC cells. 300,000 CD34+ cells isolated from mobilized peripheral blood were transduced with a multiplicity of infection (MOI) of 50. The transduction delivered a GFP- expressing transgene. During the stimulation, transduction, and recovery/expansion phases, CD34+ cells were exposed to cytokines (SCF, TPO, Flt3-L) during the stimulation and transduction stages followed by recovery in direct co-culture with E4ORF1+ UVEC cells (C/C/E), and in the presence of E4ORF1+ UVEC cells during all 3 stages (E/E/E). Flow cytometry was used to quantify the number of cells. Fluorescent antibodies to identify CD34 and CD45 on hematopoietic stem and progenitor cells were used to determine the HSPC content of the cultures. GFP expression denoted successfully transduced cells. After quantification via flow cytometry and hemocytometer counts, total CD45+ CD34+ HSPC yield is presented in the bar graph in **Fig. 14A****,** with successfully transfected CD45+ CD34+ HSPC yield presented in the bar graph in **Fig. 14B** (axes show numbers of cells in millions). All conditions not specified herein are specified in the Examples.

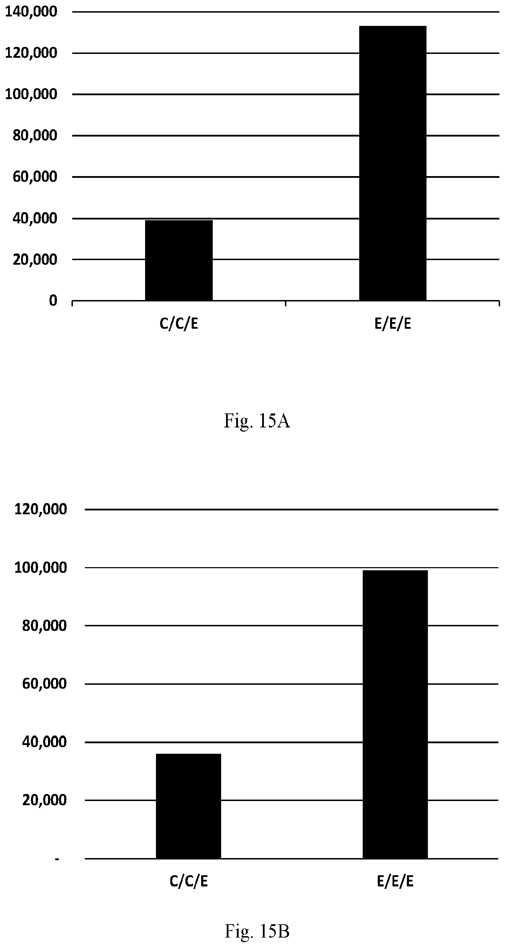

**Fig. 15A-B.** Total CD45+ CD34+ CD38- CD45RA- CD49f+ stem cell yield is increased during lentiviral transduction with the use of E4ORF1+ UVEC cells. 300,000 CD34+ cells isolated from mobilized peripheral blood were transduced with a multiplicity of infection (MOI) of 50. The transduction delivered a green fluorescent protein (GFP) expressing transgene. During the stimulation, transduction, and recovery/expansion phases, CD34+ cells were exposed to cytokines (SCF, TPO, Flt3-L) during the stimulation and transduction stages followed by recovery in direct co-culture with E4ORF1+ UVEC cells (C/C/E), and in the presence of E4ORF1+ UVEC cells during all 3 stages (E/E/E). Flow cytometry was used to quantify the number of cells. Fluorescent antibodies to identify CD38, CD45RA, CD49f, CD34 and CD45 on hematopoietic stem cells were used to determine the stem cell content of the cultures. GFP expression denoted successfully transduced cells. After quantification via flow cytometry and hemocytometer counts, total stem cell yield is presented as a bar graph in **Fig. 15A****,** with successfully transduced stem cell yield presented as a bar graph in **Fig. 15B**(axes show numbers of cells). All conditions not specified herein are specified in the Examples.

### DETAILED DESCRIPTION

The "Summary of the Invention," "Figures," "Brief Description of the Figures," "Examples," and "Claims" sections of this patent disclosure describe some of the main embodiments of the invention. This "Detailed Description" section provides certain additional description relating to the methods of the present invention and compositions of the disclosure, and is intended to be read in conjunction with all other sections of this patent disclosure. Furthermore, and as will be apparent to those in the art, the different embodiments described throughout this patent disclosure can be, and are intended to be, combined in various different ways.

Certain definitions are provided below. Other terms are either defined elsewhere in this patent disclosure, have a meaning that is clear from the context in which they are used, or are used in accordance with their usual meaning in the art.

As used herein, the terms "about" and "approximately," when used in relation to numerical values, mean within + or - 20% of the stated value.

The term "culturing" as used herein, refers to the propagation of cells on or in media of various kinds. "Co-culturing" refers to the propagation of two or more distinct types of cells on or in media of various kinds, for instance, in some embodiments, E4ORF1+ ECs and hematopoietic stem or progenitor cells (HSPCs) may be co-cultured.

The term "engineered" when used in relation to cells herein refers to cells that have been engineered by man to result in the recited phenotype (e.g. E4ORF1⁺), or to express a recited nucleic acid molecule or polypeptide. The term "engineered cells" is not intended to encompass naturally occurring cells, but is, instead, intended to encompass, for example, cells that comprise a recombinant nucleic acid molecule, or cells that have otherwise been altered artificially (e.g. by genetic modification), for example so that they express a polypeptide that they would not otherwise express, or so that they express a polypeptide at substantially higher levels than that observed in non-engineered endothelial cells.

The terms "expansion" or "expanding" as used herein in the context of cells or cell culture refer to an increase in the number of cells of a certain type (for example "target cells," such as HSPCs) from an initial population of cells. The initial cells used for expansion need not be the same as the cells generated as a result of the expansion. For instance, the expanded cells may be produced by growth and/or differentiation of the initial population of cells.

"Genetic modification" or "gene-modified" refers to any addition, deletion or disruption of or to a cell's normal nucleotide sequences, and includes, but is not limited to, "gene-editing." Genetic modification is typically performed using a "gene delivery" method.

"Gene delivery" refers to delivery of any exogenous nucleic acid molecule to a cell. Gene delivery methods encompassed by the present invention include, but are not limited to, "transduction" methods (i.e. virus-mediated transfer of nucleic acid to a target cell), and "transfection" methods (i.e. non-virus-mediated transfer of nucleic acid to a target cell).

The term "hematopoietic stem cell" or "HSC" refers to a clonogenic, self-renewing pluripotent cell capable of ultimately differentiating into all cell types of the hematopoietic system, including B cells T cells, NK cells, lymphoid dendritic cells, myeloid dendritic cells, granulocytes, macrophages, megakaryocytes, and erythroid cells. As with other cells of the hematopoietic system, HSCs are typically defined by the presence of a characteristic set of cell markers.

The term "hematopoietic stem or progenitor cell" or "HSPC," as used herein, encompasses HSCs, as defined above, as well as multipotent non-self-renewing progenitor cells that are capable of ultimately differentiating into all cell types of the hematopoietic system, and oligopotent and unipotent progenitor cells capable differentiating into certain cell types of the hematopoietic system. HSPCs include CMPs, MPs, MEPs, and GMPs, each of which is defined elsewhere herein.

As used herein the term "isolated" refers to a product (e.g. cells) or composition which is separated from at least one other product or composition with which it is associated in its naturally occurring state, whether in nature or as made synthetically.

As used herein, the term "recombinant" refers to nucleic acid molecules that are generated by man (including by a machine) using methods of molecular biology and genetic engineering (such as molecular cloning), and that comprise nucleotide sequences that would not otherwise exist in nature. Thus, recombinant nucleic acid molecules are to be distinguished from nucleic acid molecules that exist in nature - for example in the genome of an organism. A nucleic acid molecule that comprises a complementary DNA or "cDNA" copy of an mRNA sequence, without any intervening intronic sequences such as would be found in the corresponding genomic DNA sequence, would thus be considered a recombinant nucleic acid molecule. By way of example, a recombinant E4ORF1 nucleic acid molecule might comprise an E4ORF1 coding sequence operatively linked to a promoter and/or other genetic elements with which that coding sequence is not ordinarily associated in a naturally-occurring adenovirus genome.

The term "self-renewal" refers to the ability of a cell to divide and generate at least one daughter cell with the identical (e.g., self-renewing) characteristics of the parent cell. The second daughter cell may commit to a particular differentiation pathway. For example, a self-renewing hematopoietic stem cell divides and forms one daughter stem cell and another daughter cell committed to differentiation in the myeloid or lymphoid pathway. A committed progenitor cell has typically lost the self-renewal capacity, and upon cell division produces two daughter cells that display a more differentiated (i.e., restricted) phenotype.

The terms "subject" and "patient" are used herein interchangeably and refer to, except where indicated, mammals such as humans and non-human primates, as well as rabbits, rats, mice, goats, pigs, and other mammalian species.

The phrase "substantially pure" as used herein in relation to a cell population refers to a population of cells of a specified type (e.g. as determined by expression of one or more specified cell markers, morphological characteristics, or functional characteristics), or of specified types (plural) in embodiments where two or more different cell types are used together, that is at least about 50%, preferably at least about 75-80%, more preferably at least about 85-90%, and most preferably at least about 95% of the cells making up the total cell population. Thus, a "substantially pure cell population" refers to a population of cells that contain fewer than about 50%, preferably fewer than about 20-25%, more preferably fewer than about 10-15%, and most preferably fewer than about 5% of cells that are not of the specified type or types.

Embodiments of the present invention described herein involve E4ORF1+ endothelial cells (E4ORF1+ ECs). Such cells comprise an E4ORF1-encoding nucleic acid molecule. The E4ORF1 polypeptides and/or the E4ORF1-encoding nucleic acid molecules may have amino acid sequences or nucleotide sequences that are specified herein or that are known in the art, or may have amino acid or nucleotide sequences that are variants, derivatives, mutants, or fragments of such amino acid or nucleotide sequences - provided that such a variants, derivatives, mutants, or fragments have, or encode a polypeptide that has, one or more of the functional properties described herein (which include, but are not limited to, an ability to support the maintenance or expansion of endothelial cells in culture, and/or to support the expansion of another target cell type (such as HSPCs) in culture, and/or to improve gene transfer efficiency to another target cell type (such as HSPCs).

In those embodiments involving adenovirus E4ORF1 polypeptides, the polypeptide sequence used may be from any suitable adenovirus type or strain, such as human adenovirus type 2, 3, 5, 7, 9, 11, 12, 14, 34, 35, 46, 50, or 52. In some preferred embodiments the polypeptide sequence used is from human adenovirus type 5. Amino acid sequences of such adenovirus polypeptides, and nucleic acid sequences that encode such polypeptides, are well known in the art and available in well-known publicly available databases, such as the Genbank database. For example, suitable sequences include the following: human adenovirus 9 (Genbank Accession No. CAI05991), human adenovirus 7 (Genbank Accession No. AAR89977), human adenovirus 46 (Genbank Accession No. AAX70946), human adenovirus 52 (Genbank Accession No. ABK35065), human adenovirus 34 (Genbank Accession No. AAW33508), human adenovirus 14 (Genbank Accession No. AAW33146), human adenovirus 50 (Genbank Accession No. AAW33554), human adenovirus 2 (Genbank Accession No. AP. sub. --000 196), human adenovirus 12 (Genbank Accession No. AP.sub.--000141), human adenovirus 35 (Genbank Accession No. AP.sub.--000607), human adenovirus 7 (Genbank Accession No. AP.sub.--000570), human adenovirus 1 (Genbank Accession No. AP.sub.--000533), human adenovirus 11 (Genbank Accession No. AP. sub. --000474), human adenovirus 3 (Genbank Accession No. ABB 17792), and human adenovirus type 5 (Genbank accession number D12587).

In some embodiments the endothelial cells described herein comprise E4ORF1 nucleic acid or amino acid sequences without other sequences from the adenovirus E4 region - for example, in certain embodiments the endothelial cells comprise E4ORF1 sequences but do not comprise the entire E4 region, or other ORFs from the entire E4 region -such as E4ORF2, E4ORF3, E4ORF4, and/or E4ORF5. However, in other embodiments the endothelial cells described herein may comprise E4ORF1 sequences together with one or more other nucleic acid or amino acid sequences from the E4 region, such as E4ORF2, E4ORF3, E4ORF4, E4ORF5, or E4ORF6 sequences, or variants, mutants or fragments thereof.

In all of the embodiments described herein that relate to polypeptides and/or nucleic acid molecules, in some embodiments such polypeptides and/or nucleic acid molecules have the same amino acid or nucleotide sequences as those specifically recited herein or known in the art (for example in public sequence databases, such as the Genbank database). In some embodiments the polypeptides and nucleic acid molecules disclosed herein may have amino acid or nucleotide sequences that are variants, derivatives, mutants, or fragments of such sequences, for example variants, derivatives, mutants, or fragments having greater than 85% sequence identity to such sequences. In some embodiments, the variants, derivatives, mutants, or fragments have about an 85% identity to the known sequence, or about an 88%, 89%, 90%, 91%, 92%, 93%, 94%, 95%, 96%, 97%, 98%, or 99% sequence identity to the known sequence. In some embodiments, a variant, derivative, mutant, or fragment of a known nucleotide sequence is used that varies in length by about 50 nucleotides, or about 45 nucleotides, or about 40 nucleotides, or about 35 nucleotides, or about 30 nucleotides, or about 28 nucleotides, 26 nucleotides, 24 nucleotides, 22 nucleotides, 20 nucleotides, 18 nucleotides, 16 nucleotides, 14 nucleotides, 12 nucleotides, 10 nucleotides, 9 nucleotides, 8 nucleotides, 7 nucleotides, 6 nucleotides, 5 nucleotides, 4 nucleotides, 3 nucleotides, 2 nucleotides, or 1 nucleotide relative to the known nucleotide sequence. In some embodiments, a variant, derivative, mutant, or fragment of a known amino sequence is used that varies in length about 50 amino acids, or about 45 amino acids, or about 40 amino acids, or about 35 amino acids, or about 30 amino acids, or about 28 amino acids, 26 amino acids, 24 amino acids, 22 amino acids, 20 amino acids, 18 amino acids, 16 amino acids, 14 amino acids, 12 amino acids, 10 amino acids, 9 amino acids, 8 amino acids, 7 amino acids, 6 amino acids, 5 amino acids, 4 amino acids, 3 amino acids, 2 amino acids, or 1 amino acid relative to the known amino acid sequence.

The nucleic acid molecules described herein can be used in constructs that contain various other nucleic acid sequences, genes, or coding regions, depending on the desired use, for example, antibiotic resistance genes, reporter genes or expression tags (such as, for example nucleotides sequences encoding GFP), sequences useful for homologous recombination, or any other nucleotide sequences or genes that might be desirable. The polypeptides described herein can be expressed alone or as part of fusion proteins.

In some embodiments the nucleic acid molecules described herein can be under the control of one or more promoters to allow for expression. Any promoter able to drive expression of the nucleic acid sequences in the desired cell type can be used. Examples of suitable promoters include, but are not limited to, the CMV, SV40, RSV, HIV-Ltr, and MML promoters. The promoter can also be a promoter from the adenovirus genome, or a variant thereof.

In some embodiments, the nucleic acid molecules described herein can be placed under the control of an inducible promoter, so that expression of the nucleic acid sequences can be turned on or off as desired. Any suitable inducible expression system can be used, such as, for example, a tetracycline inducible expression system, or a hormone inducible expression system. For example, the nucleic acid molecules disclosed herein can be expressed while they are needed and then switched off when the desired outcome has been achieved. The ability to turn on or turn off expression could be particularly useful for *in vivo* applications - for example for in vivo applications of the genetically modified target cells produced using the methods described herein.

The nucleic acid molecules described herein may comprise naturally occurring nucleotides, synthetic nucleotides, or a combination thereof. For example, in some embodiments the nucleic acid molecules can comprise RNA, such as synthetic modified RNA that is stable within cells and can be used to direct protein expression/production directly within cells. In other embodiments the nucleic acid molecules can comprise DNA. In embodiments where DNA is used, the DNA sequences may be operably linked to one or more suitable promoters and/or regulatory elements to allow (and/or facilitate, enhance, or regulate) expression within cells, and may be present in one or more suitable vectors or constructs.

The handling, manipulation, and expression of the polypeptides and nucleic acid molecules disclosed herein may be performed using conventional techniques of molecular biology and cell biology. Such techniques are well known in the art. For example, one may refer to the teachings of Sambrook, Fritsch and Maniatis eds., "Molecular Cloning A Laboratory Manual, 2nd Ed., Cold Springs Harbor Laboratory Press, 1989); the series Methods of Enzymology (Academic Press, Inc.), or any other standard texts for guidance on suitable techniques to use in handling, manipulating, and expressing nucleotide and/or amino acid sequences. Additional aspects relevant to the handling or expression of E4ORF1 sequences are described in U.S. Patent No. 8,465,732.

Disclosed herein are improved methods for gene delivery to target cells. In some embodiments any suitable gene delivery system known in the art can be used, such as any suitable transfection system or any suitable transduction system.

Transfection methods that can be used in accordance with the present invention include, but are not limited to, liposome-mediated transfection, polybrene-mediated transfection, DEAE dextran-mediated transfection, electroporation, nucleofection, calcium phosphate precipitation, microinjection, and micro-particle bombardment.

Transduction methods that can be used include, but are not limited to, lentivirus-mediated transduction, adenovirus-mediated transduction, retrovirus-mediated transduction, adeno-associated virus-mediated transduction and herpesvirus-mediated transduction.

In some embodiments target cells may be genetically modified so that they comprise a corrected version of a gene known to be involved in, or suspected of being involved in, a disease or disorder that affects the target cell type, or any other gene, such as a therapeutically useful gene, that it may be desired to provide in the target cells or administer or deliver using the target cells. In some embodiments the methods of the present invention may be utilized in the performance of, or in conjunction with, genome editing techniques, such as genome editing techniques that utilize engineered nucleases to insert, delete, or replace desired nucleotide sequences in the genome of a target cell. Typically such nucleases create site-specific double-strand breaks in the genome, which are repaired using non-homologous end-joining or homologous recombination. There are four main families of such nucleases that are currently used in gene editing techniques - i.e. meganucleases, zinc finger nucleases (ZFNs), Transcription Activator-Like Effector-based Nucleases or "TALENs," and CRISPR-Cas system nucleases (e.g. Cas9).

The methods of the present invention involve use of E4ORF1+ Endothelial Cells. Such endothelial cells can be derived from any suitable source of endothelial cells known in the art. For example, in some embodiments the endothelial cells are vascular endothelial cells. In some embodiments the endothelial cells are primary endothelial cells. In some embodiments the engineered endothelial cells are mammalian cells, such as human or non-human primate cells, or rabbit, rat, mouse, goat, pig, or other mammalian cells. In some embodiments the endothelial cells are primary human endothelial cells. In some embodiments the endothelial cells are umbilical vein endothelial cells (UVEC), such as human umbilical vein endothelial cells (HUVEC). Other suitable endothelial cells that can be used include those described previously as being suitable for E4ORF1-expression in U.S. Patent No. 8,465,732.

The engineered endothelial cells disclosed herein may exist in, or be provided in, various forms. For example, in some embodiments the engineered endothelial cells may comprise a population of cells, such as an isolated population of cells. In some embodiments the engineered endothelial cells may comprise a population of cells *in vitro.* In some embodiments the engineered endothelial cells may comprise a substantially pure population of cells. For example, in some embodiments at least about 50%, preferably at least about 75-80%, more preferably at least about 85-90%, and most preferably at least about 95% of the cells making up a total cell population will be engineered endothelial cells disclosed herein. In some embodiments the engineered endothelial cells may be provided in the form of a composition containing the engineered cells and one or more additional components. For example, disclosed herein is a composition comprising a population of engineered endothelial cells as described herein together with a carrier solution, such as a physiological saline solution, cell suspension medium, cell culture medium, or the like. In some embodiments such compositions may be therapeutic compositions - comprising a population of engineered endothelial cells and a carrier solution that is suitable for administration to a subject, such as a human subject. Other therapeutically acceptable agents can be included if desired. One of ordinary skill in the art can readily select suitable agents to be included in the therapeutic compositions depending on the intended use.

Methods of culturing cells are well known in the art and any suitable cell culture methods can be used. For example, E4ORF1+ cells can be cultured using methods known to be useful for culturing other endothelial cells, or, methods known to be useful for culturing E4ORF1-expressing endothelial cells, for example as described in U.S. Patent No. 8,465,732. In some embodiments the engineered endothelial cells disclosed herein can be cultured in the absence of serum, or in the absence of exogenous growth factors, or in the absence of both serum and exogenous growth factors. The engineered endothelial cells disclosed herein can also be cryopreserved. Various methods for cell culture and cell cryopreservation are known to those skilled in the art, such as the methods described in Culture of Animal Cells: A Manual of Basic Technique, 4th Edition (2000) by R. Ian Freshney ("Freshney").

The present invention involves the use of E4ORF1+ EC feeder cells to support the survival, expansion, and or genetic modification of other cells (e.g. target cells) in a co-culture method. For example, in one embodiment a population of E4ORF1+ ECs can be used as feeder cells to support the growth, expansion, or genetic modification of stem or progenitor cells, such as HSPCs and HSPCs.

Disclosed herein are co-culture methods for culturing a population of E4ORF1+ ECs and a population of target cells. Such co-culture methods may comprise culturing a population of E4ORF1+ ECs and a population of target cells together in the same culture vessel. In some such embodiments the E4ORF1+ ECs may form a feeder cell layer on a surface of the culture vessel, and the target cells may be placed on the feeder cell layer. Also disclosed herein is a method of culturing target cells comprising: contacting the target cells with conditioned medium obtained from a culture of the E4ORF1+ cells.

Also disclosed herein are kits for carrying out the various methods described herein. Such kits may contain any of the components described herein, including, but not limited to, nucleotide sequenced, vectors, endothelial cells, E4ORF1+ endothelial cells, control non-engineered endothelial cells, target cells (such as HSPCs), media or compositions useful for maintaining or expanding E4ORF1+ ECs or target cells, or any combination thereof. All such kits may optionally comprise instructions for use, containers, culture vessels and the like. A label may accompany the kit and may include any writing or recorded material, which may be electronic or computer readable form (e.g., disk, optical disc, memory chip, or tape) providing instructions or other information for use of the kit contents.

Certain aspects of the present invention may be further described in the following non-limiting Examples. It should be noted that the description in the Examples, as well as the foregoing descriptions of specific embodiments of the present invention, have been presented for purposes of illustration and description. They are not intended to be exhaustive or to limit the invention to the precise forms disclosed. Rather many modifications and variations of the specific embodiments described herein are possible in light of the teachings of this patent application. The embodiments presented herein were chosen and described in order to best explain the principles of the invention and its practical application, to thereby enable others skilled in the art to best utilize the invention and various embodiments with various modifications as are suited to the particular use contemplated.

### EXAMPLES

Some of the data generated in the below Examples is provided in the Figures. Furthermore, the Brief Description of the Figures section of this patent disclosure contains certain additional information regarding pertinent to these Examples, including additional details regarding the experiments performed and the data generated. The present Examples are intended to be read in conjunction with the Brief Description of the Figures and the Figures themselves.

### Example 1

### Effect of E4ORF1+ Co-Culture on Expansion & Viral Transduction of HSPCs

Experiments were performed to assess the effects of E4ORF1+ endothelial cells on the ability to expand and transduce CD34+/CD45+ HSPCs. CD34+ cells (Lonza) were transduced with Red Fluorescent Protein (RFP) and expanded on E4ORF1+ umbilical vein endothelial cell (UVEC) cultures in 6-well plates using the three different protocols, as follows:

In protocol 1, transduction was commenced 24 hrs prior to starting HSPC - E4ORF1+ UVEC co-culture ("Pre-transduced").

In protocol 2, transduction was performed concurrently with HSPC - E4ORF1+ UVEC co-culture ("Concurrent-transduced").

In protocol 3 HSPCs were co-cultured with E4ORF1+ UVEC for 48hrs, after which floating HSPCs were transferred to an empty well for transduction (24hrs), and then following transduction the HSPCs were co-cultured with E4ORF1+ UVEC ("Expanded-transduced").

In each of the above protocols cell culture was performed under hypoxic conditions. Also, in each protocol approximately 100,000 hematopoietic cells (of which 95,000 were determined to be CD34+ by flow cytometry) were plated either in an empty well (protocol 1) or in a well that had been pre-seeded to confluency with E4ORF1+ UVEC cells (protocols 2 and 3). The number of E4ORF1+ UVEC cells present at confluency of one well of a six-well plate is about 300,000 cells. The media consisted of StemMACS HSC expansion media (manufactured by Miltenyi Biotec) plus 100ng/ml of SCF, Flt3, and TPO cytokines. The transduction event consisted of a 24-hr period during which the cells were incubated with lentivirus engineered to RFP and polybrene. The transduced cells were then collected, spun down, suspended in fresh media with cytokines, and re-plated onto confluent E4ORF1+ UVEC cultures. The culture medium (including cytokines) was changed every two days thereafter. In all three protocols cells were expanded for a total of 8 days, at which time the cells were collected, counted, and stained with antibodies to CD34 and CD45 for FACS analysis.

Both total hematopoietic cells and the CD34+ sub-population (i.e. HSPCs) demonstrated a higher fold expansion when the transduction process took place in the setting of a co-culture with E-CEL UVEC cells compared to transducing the CD34+ cells alone **(****Fig. 1****).** Furthermore, the expansion potential of these cells further increased following a 48hr period of co-culture prior to initiating the transduction process. Similarly, the most efficient transduction process proved to be that in which hematopoietic cells were co-cultured with E4ORF1+ UVEC cells for 48hrs prior to commencing the transduction procedure **(****Fig. 2****).** Importantly, these results indicate that the CD34+ subpopulation in particular both was most effectively transduced **(****Fig. 2A****)** and showed the greatest expansion of the transduced population **(****Fig. 2B****).** Taken together, these data suggest that allowing the CD34+ hematopoietic cells to acclimate to co-culture with E4ORF1+ UVEC cells dramatically increases both the efficiency of transduction and the overall cellular output of transduced cell populations.

### Example 2

### Effect of Floating Fraction & Cytokines on Viral Transduction Efficiency

Experiments were performed to determine the effect of removing the 'floating fraction' of CD34+ hematopoietic cells on transduction efficiency and to determine if cytokine concentration has an influence on transduction efficiency.

CD34+ cells (Lonza) were transduced with Blue Fluorescent Protein (BFP) and expanded on E4ORF1+ UVEC cultures in 6-well plates using the following 4 different protocols, as follows:

In protocols 1 and 3, HSPCs were expanded on E4ORF1+ UVEC cultures for 96 hours and then subsequently transduced by incubating the HSPCS with lentivirus engineered to express BFP for 24 hours - while the HSPCs were still on the E4ORF1+ EC feeder layer. The HSPCs were then expanded on the E4ORF1+ UVEC cultures for a further two days. Results obtained using these protocols are referred to in the figures as "co-transduction" results

In protocols 2 and 4, HSPCs were expanded on E4ORF1+ UVEC cultures for 96 hours and then subsequently the floating fraction was transferred to an empty well and was transduced by incubating the HSPCs with lentivirus engineered to express BFP for 24 hours. After the transduction period the HSPCs were cultured on E4ORF1+ UVEC cultures for another two days. Results obtained using these protocols are referred to in the figures as "separated transduction" results.

The cell culture media used in protocols 1 and 2 consisted of StemMACS HSC expansion media (manufactured by Miltenyi Biotec) with100ng/ml each of the cytokines SCF, Flt3, and TPO - referred to in the figures as "norm cyto dose." The cell culture media used in protocols 3 and 4 consisted of StemMACS HSC expansion media (manufactured by Miltenyi Biotec) with 300ng/ml each of SCF and Flt3, 100ng/ml each of TPO and IL-6, and 20ng/ml of IL-3 - referred to in the figures as "high cyto dose".

In each of the above protocols all cell culture was performed under hypoxic conditions. Also, in each of these 4 protocols 100,000 hematopoietic cells (comprising 95,000 CD34+ cells - as determined by flow cytometry) were plated on a confluent layer of E4ORF1+ UVEC cells. The number of E4ORF1+ UVEC cells present at confluency of one well of a six-well plate is about 300,000 cells. After a 96hr period of hematopoietic cell / E4ORF1+ EC co-culture, the transduction event was initiated which consisted of a 24-hr period during which the samples were incubated with a lentivirus engineered to express BFP and polybrene. The transduced samples were then collected, spun down, re-suspended in fresh media (with cytokines - as indicated), and plated onto E4ORF1+ UVEC cultures. Media (with cytokines) was exchanged every two days thereafter. The expansions for all samples lasted for a total of 7 days, after which time the cells were collected, counted, and stained with antibodies to CD34 and CD45 for FACS analysis.

As expected, both the total hematopoietic cell population and the CD34+ hematopoietic cell sub-population demonstrated a higher fold expansion when a higher cytokine dose was used **(****Fig. 3****).** There appeared to be little difference in the fold expansion of hematopoietic cell populations transduced in the presence of E4ORF1+ UVEC cultures as compared to those transduced in the absence of E4ORF1+ ECs. Surprisingly, however, the efficiency of transduction was greater when transduction was performed in the presence of the E4ORF1+ UVEC cells than when transduction was performed on hematopoietic cells that had been temporarily separated from the E4ORF1 UVEC cultures during the transduction event **(****Fig. 4****).** Thus, despite the presence of a second cell-type (i.e. the E4ORF1+ UVEC) during the transduction step that could potentially have absorbed/sequestered virus, hematopoietic cells - and in particular the CD34+ / HSPC cell fraction - still demonstrated a higher capacity for viral intake when transduced in the presence of E4ORF1+ UVEC cells. While cytokine dosing had little effect on transduction efficiency **(****Fig. 4****),** utilizing a higher cytokine dose did result in a larger number of transduced CD34+ cells overall - which may be due to an increase in cell expansion and/or an increase in the number of transduced CD34+ CD45+ cells **(****Fig. 5****).** Taken together, these results suggest that transducing HSPCs in the presence of E4ORF1+ UVEC cultures can improve viral transduction efficiencies, and that the number of transduced cells can be further enhanced by increasing the cytokine dose used during expansion.

The data presented in Examples 1 and 2 demonstrates that that co-culture of certain target cells, such as HSPCs, with E4ORF1+ ECs can significantly improve gene transfer efficiencies. Specifically, the results presented herein demonstrate that viral transduction of HSPCs - a major hurdle in the gene-editing field - can be significantly improved using an E4ORF1+ EC co-culture system. The presence of E4ORF1+ UVEC cells increases both the overall yield of CD34+/CD45+ HSPCs, and the efficiency of transduction in this important stem cell population. This dual benefit of E4ORF1+ EC co-culture could represent a novel means for improving the efficiency of a variety of gene transfer methods - for example for the purposes of gene editing and/or gene therapy.

### Example 3

### Rescue of Transfected Cells by Subsequent EC Co-Culture

Transfected or transduced cells, such as cells transfected by electroporation, can be cultured with ECs after transfection or transduction. Such subsequent co-culture can "rescue" cells that have been damaged by the transfection or transduction process -reducing cell death and loss of the transfected or transduced cells. Such co-culturing should be commenced "immediately" after the transfection or transduction step is commenced (as that term is defined in this patent specification), and should continue for sufficient time to allow recovery of the target cells. Without wishing to be bound by theory, it is believed that such "rescue" can occur as a result of cell-to-cell contact between the two cell populations (i.e. the transduced/transfected cells and the ECs), and/or as a result of exposure of the transduced/transfected cells to soluble factors secreted by the ECs, such as angiocrine factors and cytokines. The co-culture of the two cell populations can also facilitate and/or increase expansion of the transduced or transfected cells.

The ECs used can be any desired ECs. Organ-specific ECs (i.e. ECs derived from the same organ that the transduced/transfected cells are derived from) can be used. E4ORF1+ ECs can also be used.

### Example 4

### Effect of E4ORF1+ Co-Culture on Expansion & Transfection of HSPCs by Electroporation

Goal: To test the effect of an electroporation system (Lonza 4D-Nucleofector system) for transfecting plasmid GFP into human mobilized peripheral blood (mPB) CD34+ cells in the context of co-culture with E4ORF1+ endothelial cells.

Experimental Design: Cell culturing was performed at 5% Oxygen and 37° unless otherwise noted. During the stimulation, transfection, and recovery/expansion phases, CD34+ cells were exposed to cytokines (SCF, TPO, Flt3-L) alone (denoted as C/C/C), cytokines during the stimulation and transfection stages followed by recovery in direct co-culture with E4ORF1+ UVEC cells (C/C/E), or in the presence of E4ORF1+ UVEC cells during all 3 stages - i.e. the stimulation stage, the transfection stage, and the recovery stage (E/E/E). On Day 0, 1,500,000 human mPB CD34+ cells (from AllCells) were plated into two wells of a low-binding attachment 6-well dish (500K per well) and in 1 well of pre-conditioned E4ORF1+ UVEC 6-well dish (500K), all in StemMACS HSC expansion media (manufactured by Miltenyi Biotec) with cytokines (100ng/ml SCF, Flt3, and TPO). On Day 1 (24 hrs later), all of the samples were collected, spun down, and counted. The cells will be resuspended in Nucleofector Solution and maxGFP plasmid (from Lonza). The samples were electroporated, and plated as follows: #1: the first low-bind well went into a new low-bind well (C/C/C). #2: the second low-bind well went into an E4ORF1+ UVEC well (C/C/E). #3: the E4ORF1+ UVEC well went into a second E-CELE4ORF1+ UVEC well (E/E/E). All samples continued to be cultured in StemMACS HSC expansion media (manufactured by Miltenyi Biotec) with cytokines (100ng/ml SCF, Flt3, and TPO). Plates were then placed in a 5% Oxygen incubator at 37° for 96 hours (fed again with fresh media and cytokines at 48 hours post-transfection), at which time they were analyzed via cell counts, flow cytometry, and CFU assays. In each case where there were E4ORF1+ ECs used, there were approximately 300,000 of such cells present in each well - i.e. the co-culture and transfection/transduction was performed in the presence of a confluent monolayer of E4ORF1+ ECs (the number of ECs at confluency of one well of a 6-well plate is about 300,000).

Results: Total hematopoietic cells **(****Fig. 6****)** and specifically CD34+ cells **(****Fig. 7A****)** expanded on ECs for the entirety of the assay showed the greatest overall expansion (6.4-fold increase), followed by CD34+ cells expanded on ECs just after the transfection event (2.7-fold increase). CD34+ cells transfected and expanded in the absence of ECs entirely showed the poorest expansion (0.7-fold increase). Notably, transfection efficiency of the CD34+ population was not adversely affected due to the presence of ECs (78% CD34+ cells transfected in E/E/E samples, vs 73% CD34+ transfected and 76% CD34+ transfected in C/C/C and C/C/E samples, respectively with overall yields of transfected CD34+ notably higher with increased exposure to E4ORF1+ UVEC cells - **(****Fig. 7B****).** While all samples were positive for total colony formation in CFU assays **(****Fig. 8A****),** only CD34+ cells that were expanded in the presence of ECs showed GEMM formation **(****Fig. 8D** and **Fig. 9****),** indicative of multi-potent progenitors (MMPs) being present in those expanded populations.

Conclusions: Transfection and subsequent expansion of CD34+ cells in the presence of ECs E4ORF1+ UVEC cells results in the greatest yield of total cells and transfected cells, as compared to delayed intervention or complete omission of the EC population during the process. Furthermore, transfection efficiency is surprisingly not adversely affected by the presence of the EC population during the transfection event. Lastly, the presence of GEMM colony formation in CFU assays on samples co-cultured with ECs demonstrates that the ECs play an important role in maintaining the MPP population of CD34+ cells during the expansion process. These results indicate that introducing E4ORF1+ UVEC cells when using this transfecting hematopoetic cells may provide improvements as compared to gene-delivery performed in the absence of such cells.

### Example 5

### Transduction of monkey non-human primate mPB CD34+ with lenti-GFP using the E4ORF1+ UVEC platform

Goal: To assess the transduction efficiency and expansion of non-human primate mPB CD34+ in the context of E4ORF1+ UVEC co-culture.

Experimental Design: Expansion assays were performed at 5% Oxygen. During the stimulation, transfection, and recovery/expansion phases, CD34+ cells were exposed to cytokines (SCF, TPO, Flt3-L) alone (denoted as C/C/C), cytokines during the stimulation and transfection stages followed by recovery in direct co-culture with E4ORF1+ UVEC cells (C/C/E), or in the presence of E4ORF1+ UVEC cells during all 3 stages (E/E/E). 450,000 non-human primate mPB CD34+ cells were each plated as follows: #1) into one retronectin coated well (6-well format); #2) into one retronectin coated well (6-well format); #3) into one confluent E4ORF1+ UVEC cell well (6-well format). The initial CD34+ plating of all three samples occurred on Day 0, and was done in StemMACS HSC expansion media (manufactured by Miltenyi Biotec) with 100ng/ml of SCF, Flt3, and TPO. On Day 1 (24hrs later), all samples were re-fed with fresh media and cytokines; additionally, GFP lenti-virus at an MOI of 25 was added to samples all three samples. On Day 2 (24hrs post-transduction), sample #1 was passed into a new retronectin coated well. Samples #2 and #3 were passed into one new well each previously seeded with E4ORF1+ UVEC cells. All samples were re-fed in fresh media and cytokines. On Day 4 (72 hrs post-transduction) all samples were re-fed with fresh media and cytokines. On Day 6 (120 hrs post-transduction) the cells were passed to either new retronectin coated wells (sample #1) or E4ORF1+ UVEC cell wells (samples #2 and #3) in fresh media and cytokines. On Day 8 (168 hrs post-transfection) all samples were refed with fresh media and cytokines. On Day 10 (192 hrs post-transfection) all samples were collected for cell counts and staining (for FACS).. In each case where there were E4ORF1+ ECs used, there were approximately 300,000 of such cells present in each well - i.e. the co-culture and transfection/transduction was performed in the presence of a confluent monolayer of E4ORF1+ ECs (the number of ECs at confluency of one well of a 6-well plate is about 300,000).

Results: Total hematopoietic cells **(****Fig. 10****)** and specifically CD34+ cells **(****Fig. 11** left) expanded on ECs for the entirety of the assay showed a higher overall expansion (62-fold increase) as compared to CD34+ cells expanded on ECs just after transducing with GFP (48-fold increase). CD34+ cells transduced with GFP and expanded in the absence of ECs entirely showed the poorest expansion (7-fold increase). Transduction efficiency of the CD34+ population **(****Fig. 11****)** was slightly decreased in the presence of ECs (42% CD34+ cells transfected in E/E/E samples, vs 60% CD34+ transfected in C/C/C samples). Interestingly, CD34+ cells transduced in the absence of ECs but then subsequently co-cultured on ECs also showed a slight decrease in transduction efficiency (51% CD34+ transfected in C/C/E samples). However, overall yields of CD34+ transfected cells were higher when co-cultured with E4ORF1+ UVEC cells (**Fig. 11** **B**). A similar trend was noted for the transduction efficiency of the phenotypic stem cell population (measured by CD45+CD34+CD38-CD45RA-CD49f+ cells): E/E/E sample - 21%; C/C/E sample - 24%; C/C/C sample - 38%. **Fig. 12****).** Notably, there was a significant increase in the number of transduced stem cells **(Fig. 12B)** in the E/E/E sample (-900,000 cells) and C/C/E sample (-850,000 cells) compared to the C/C/C sample (-300,000).

Conclusions: While the efficiency of transduction of monkey CD34+ cells and, more specifically, CD34+ stem cells was slightly less when ECs were present during the transduction event, the slight decrease in transduction was counterbalanced by the noticeable increase in the number of these cells in the expanded product when ECs were part of the process. Therefore, by altering the conditions of the transduction event, via MOI increase for example, an increase in transduction efficiency may be attained while still maintaining the significant increase in overall yield of cells that the E4ORF1+ UVEC cell platform affords.

### Example 6

### Transduction of human mPB CD34+ with lenti-GFP using the E4ORF1+ UVEC cell platform.

Goal: To determine the effects on transduction efficiency and/or expansion potential of human mPB CD34+ when transduced with lenti GFP in the presence of E-E4ORF1+ UVEC cells.

Experimental Design: Expansion assays were performed at 5% Oxygen. During the stimulation, transfection, and recovery/expansion phases, CD34+ cells were exposed to cytokines (SCF, TPO, Flt3-L) alone (denoted as C/C/C), cytokines during the stimulation and transfection stages followed by recovery in direct co-culture with E4ORF1+ UVEC cells (C/C/E), or in the presence of E4ORF1+ UVEC cells during all 3 stages (E/E/E). 300,000 human mPB CD34+ cells (from AllCells) were each plated as follows: #1) into one retronectin coated well (6-well format); #2) into one confluent E4ORF1+ UVEC cell well (6-well format). The initial CD34+ plating of samples #1 and #2 occurred on Day 0, and were done in StemMACS HSC expansion media (manufactured by Miltenyi Biotec) with 100ng/ml of SCF, Flt3, and TPO. On Day 1 (24hrs later), both samples were re-fed with fresh media and cytokines; additionally, GFP virus at an MOI of 50 was added to samples #1 and #2. On Day 2 (24hrs post-transduction), each sample was passed onto fresh E4ORF1+ UVEC cells in fresh media and cytokines. On Day 4 (72 hrs post-transduction) the cells were re-fed with fresh media and cytokines. On Day 6 (120 hrs post-transduction) the cells were collected for counting and staining (FACS). In each case where there were E4ORF1+ ECs used, there were approximately 300,000 of such cells present in each well - i.e. the co-culture and transfection/transduction was performed in the presence of a confluent monolayer of E4ORF1+ ECs (the number of ECs at confluency of one well of a 6-well plate is about 300,000).

Results: Total hematopoietic cells **(****Fig. 13****)** and specifically CD34+ cells **(****Fig. 14A****)** expanded on ECs for the entirety of the assay showed a higher overall expansion (21.5-fold increase) as compared to CD34+ cells expanded on ECs just after transducing with GFP (7.9-fold increase). Transduction efficiency of the CD34+ population was slightly decreased due to the presence of ECs (61% CD34+ cells transfected in E/E/E samples, vs. 81% CD34+ transfected in C/C/E samples - **Fig. 14B****).** Similarly, the transduction efficiency of the phenotypic stem cell population (measured by CD45+CD34+CD38-CD45RA-CD49f+ cells) was also slightly lower in the E/E/E sample (74%) vs the C/C/C sample (92%) **(****Fig. 15****).** Notably, though, there was a 3-fold increase of transduced stem cells in the E/E/E sample compared to the C/C/E sample **(****Fig. 15B****).**

Conclusions: Although the efficiency of lenti-mediated transduction of CD34+ stem cells was slightly less when ECs were present during the transduction event (74% for E/E/E vs 92% for C/C/E), this modest decrease was offset by the vast increase of successfully transduced stem cells that were generated at the conclusion of the expansion process (-100,000 transduced stem cells E/E/E vs -36,000 transduced stem cells C/C/E). Still, while high transduction efficiency (e.g. >70%) and high yield of cells are the ultimate goal of this platform, further manipulations of the transduction conditions could serve to improve transduction efficiencies further (e.g. > 90%) while not inhibiting the subsequent expansion of this vital population of cells.

The present invention is further described by the following claims.

## Claims

1. An improved method of genetically modifying CD34+ hematopoietic stem or progenitor cells (HSPCs), the method comprising:
(a) co-culturing CD34+ HSPCs cells with E4ORF1+ endothelial cells, and
(b) contacting the HSPCs with one or more exogenous nucleic acid molecules,
wherein the co-culturing step is commenced either: prior to, concurrently with, or after contacting the HSPCs with the one or more exogenous nucleic acid molecules,
thereby genetically modifying the CD34+ HSPCs.

2. The method of claim 1, wherein the HSPCs are derived from bone-marrow, peripheral blood, or umbilical cord blood.

3. The method of claim 1, wherein the endothelial cells are human endothelial cells.

4. The method of claim 1, wherein the endothelial cells are umbilical vein endothelial cells.

5. The method of claim 1, wherein the endothelial cells are human umbilical vein endothelial cells.

6. The method of claim 1, wherein the endothelial cells are mitotically inactivated.

7. The method of claim 1, wherein the step of contacting the target cells with one or more exogenous nucleic acid molecules is performed by transfection.

8. The method of claim 7, wherein the transfection comprises liposome-mediated transfection, polybrene-mediated transfection, DEAE dextran-mediated transfection, electroporation, nucleofection, calcium phosphate precipitation, microinjection, or micro-particle bombardment.

9. The method of claim 1, wherein the step of contacting the target cells with one or more exogenous nucleic acid molecules is performed by transduction.

10. The method of claim 9, wherein the transduction is performed using lentivirus-mediated transduction, adenovirus-mediated transduction, retrovirus-mediated transduction, adeno-associated virus-mediated transduction or herpesvirus-mediated transduction.

11. The method of claim 1, wherein the nucleic acid molecule is present in a plasmid vector.

12. The method of claim 1, wherein the nucleic acid molecule is present in a viral vector.

13. The method of any of the preceding claims, wherein the co-culturing step is commenced at least 48 hours prior to contacting the HSPCs with the one or more exogenous nucleic acid molecules.

14. The method of any of claims 1-6 or 9-13, wherein the HSPCs are transduced with the one or more exogenous nucleic acid molecules, and wherein the percentage of the CD34+ HSPCs that is transduced with the one or more exogenous nucleic acid molecules is increased as compared to the percentage of CD34+ HSPCs that is transduced when CD34+ HSPCs are not co-cultured with E4ORF1+ endothelial cells.

15. The method of any of claims 1-8 or 11-13, wherein the HSPCs are transfected with the one or more exogenous nucleic acid molecules, and wherein the percentage of the CD34+ HSPCs that is transfected with the one or more exogenous nucleic acid molecules is increased as compared to the percentage of CD34+ HSPCs that is transfected when CD34+ HSPCs are not co-cultured with E4ORF1+ endothelial cells.

## Patentansprüche

1. Verbessertes Verfahren zur gentechnischen Veränderung hämatopoetischer CD34+-Stamm- oder - Vorläuferzellen (CD34+ Hematopoietic Stern or Progenitor Cells, HSPC), wobei das Verfahren Folgendes umfasst:
(a) gemeinsames Kultivieren von CD34+-HSPC-Zellen mit E4ORF1+-Endothelzellen und
(b) In-Kontakt-Bringen der HSPC mit einem oder mehreren exogenen Nukleinsäuremolekülen,
wobei mit dem Schritt des gemeinsamen Kultivierens entweder vor, gleichzeitig mit oder nach dem In-Kontakt-Bringen der HSPC mit den ein oder mehreren exogenen Nukleinsäuremolekülen begonnen wird,
wodurch die CD34+-HSPC gentechnisch verändert werden.

2. Verfahren nach Anspruch 1, wobei die HSPC aus Knochenmark, peripherem Blut oder Nabelschnurblut stammen.

3. Verfahren nach Anspruch 1, wobei es sich bei den Endothelzellen um menschliche Endothelzellen handelt.

4. Verfahren nach Anspruch 1, wobei es sich bei den Endothelzellen um Endothelzellen der Nabelschnurvene handelt.

5. Verfahren nach Anspruch 1, wobei es sich bei den Endothelzellen um Endothelzellen der menschlichen Nabelschnurvene handelt.

6. Verfahren nach Anspruch 1, wobei die Endothelzellen mitotisch inaktiviert sind.

7. Verfahren nach Anspruch 1, wobei der Schritt des In-Kontakt-Bringens der Zielzellen mit einem oder mehreren exogenen Nukleinsäuremolekülen über Transfektion erfolgt.

8. Verfahren nach Anspruch 7, wobei die Transfektion durch Liposomen vermittelte Transfektion, durch Hexadimethrinbromid (Polybrene) vermittelte Transfektion, durch DEAE-Dextran vermittelte Transfektion, Elektroporation, Nukleofektion, Calciumphosphatpräzipitation, Mikroinjektion oder Mikropartikelbeschuss umfasst.

9. Verfahren nach Anspruch 1, wobei der Schritt des In-Kontakt-Bringens der Zielzellen mit einem oder mehreren exogenen Nukleinsäuremolekülen über Transduktion erfolgt.

10. Verfahren nach Anspruch 9, wobei die Transduktion unter Verwendung von durch Lentivirus vermittelter Transduktion, durch Adenovirus vermittelter Transduktion, durch Retrovirus vermittelter Transduktion, durch adeno-assoziiertes Virus vermittelter Transduktion oder durch Herpesvirus vermittelter Transduktion erfolgt.

11. Verfahren nach Anspruch 1, wobei das Nukleinsäuremolekül in einem Plasmidvektor vorliegt.

12. Verfahren nach Anspruch 1, wobei das Nukleinsäuremolekül in einem Virusvektor vorliegt.

13. Verfahren nach einem der vorhergehenden Ansprüche, wobei mit dem Schritt des gemeinsamen Kultivierens wenigstens 48 Stunden vor dem In-Kontakt-Bringen der HSPC mit den ein oder mehreren exogenen Nukleinsäuremolekülen begonnen wird.

14. Verfahren nach einem der Ansprüche 1-6 oder 9-13, wobei die HSPC mit den ein oder mehreren exogenen Nukleinsäuremolekülen transduziert werden und wobei der Prozentsatz der CD34+-HSPC, die mit den ein oder mehreren exogenen Nukleinsäuremolekülen transduziert sind, im Vergleich zum Prozentsatz von CD34+-HSPC, die transduziert sind, wenn CD34+-HSPC nicht gemeinsam mit E4ORF1+-Endothelzellen kultiviert werden, erhöht ist.

15. Verfahren nach einem der Ansprüche 1-8 oder 11-13, wobei die HSPC mit den ein oder mehreren exogenen Nukleinsäuremolekülen transfiziert werden und wobei der Prozentsatz der CD34+-HSPC, die mit den ein oder mehreren exogenen Nukleinsäuremolekülen transfiziert sind, im Vergleich zum Prozentsatz von CD34+-HSPC, die transfiziert sind, wenn CD34+-HSPC nicht gemeinsam mit E4ORF1+-Endothelzellen kultiviert werden, erhöht ist.

## Revendications

1. Procédé amélioré de modification génétique de cellules de la souche hématopoïétique ou progénitrices CD34+ (HSPC), le procédé comprenant :
(a) une culture conjointe de cellules HSPC CD34+ avec des cellules endothéliales E4ORF1+, et
(b) une mise en contact des HSPC avec une ou plusieurs molécule(s) d'acide nucléique exogène(s),
dans lequel l'étape de culture conjointe est commencée soit : avant, simultanément ou après la mise en contact des HSPC avec la ou les molécule (s) d'acide nucléique exogène(s),
en modifiant génétiquement de ce fait les HSPC CD34+.

2. Procédé de la revendication 1, dans lequel les HSPC sont dérivées de la moelle osseuse, du sang périphérique ou du sang ombilical.

3. Procédé de la revendication 1, dans lequel les cellules endothéliales sont des cellules endothéliales humaines.

4. Procédé de la revendication 1, dans lequel les cellules endothéliales sont des cellules endothéliales de la veine ombilicale.

5. Procédé de la revendication 1, dans lequel les cellules endothéliales sont des cellules endothéliales de la veine ombilicale humaine.

6. Procédé de la revendication 1, dans lequel les cellules endothéliales sont inactivées d'un point de vue mitotique.

7. Procédé de la revendication 1, dans lequel l'étape de la mise en contact des cellules cibles avec une ou plusieurs molécule(s) d'acide nucléique exogène(s) est effectuée par transfection.

8. Procédé de la revendication 7, dans lequel la transfection comprend une transfection par médiation de liposomes, une transfection par médiation de polybrène, une transfection par médiation de DEAE-dextrane, une électroporation, une nucléofection, une précipitation au phosphate calcique, une micro-injection ou un bombardement de microparticules.

9. Procédé de la revendication 1, dans lequel l'étape de la mise en contact des cellules cibles avec une ou plusieurs molécule(s) d'acide nucléique exogène(s) est effectuée par transduction.

10. Procédé de la revendication 9, dans lequel la transduction est effectuée en utilisant une transduction par médiation de lentivirus, une transduction par médiation d'adénovirus, une transduction par médiation de rétrovirus, une transduction par médiation de virus adéno-associés ou une transduction par médiation de virus herpès.

11. Procédé de la revendication 1, dans lequel la molécule d'acide nucléique est présente dans un vecteur plasmidique.

12. Procédé de la revendication 1, dans lequel la molécule d'acide nucléique est présente dans un vecteur viral.

13. Procédé de l'une quelconque des revendications précédentes, dans lequel l'étape de culture conjointe est commencée au moins 48 heures avant la mise en contact des HSPC avec la ou les molécule (s) d'acide nucléique exogène(s).

14. Procédé de l'une quelconque des revendications 1-6 ou 9-13, dans lequel les HSPC sont transduites avec la ou les molécule(s) d'acide nucléique exogène(s) et dans lequel le pourcentage des HSPC CD34+ qui est transduit avec la ou les molécule(s) d'acide nucléique exogène(s) est accru en comparaison avec le pourcentage des HSPC CD34+ qui est transduit quand les HSPC CD34+ ne sont pas cultivées conjointement avec des cellules endothéliales E4ORF1+.

15. Procédé de l'une quelconque des revendications 1-8 ou 11-13, dans lequel les HSPC sont transfectées avec la ou les molécule(s) d'acide nucléique exogène(s) et dans lequel le pourcentage des HSPC CD34+ qui est transfecté avec la ou les molécule(s) d'acide nucléique exogène(s) est accru en comparaison avec le pourcentage des HSPC CD34+ qui est transfecté quand les HSPC CD34+ ne sont pas cultivées conjointement avec des cellules endothéliales E4ORF1+.
